# EUROPEAN PATENT SPECIFICATION

(11) **EP 3 455 758 B1**
(45) Date of publication and mention of the grant of the patent: **13.08.2025**
(21) Application number: 17795744.6
(22) Date of filing: 10.05.2017
(51) Int. Cl.: G16H 10/60, G16H 50/20

(54) **SYSTEM, METHOD AND COMPUTER PROGRAM PRODUCT FOR PROVIDING FEEDBACK RELATING TO A MEDICAL EXAMINATION**
SYSTEM, VERFAHREN UND COMPUTERPROGRAMMPRODUKT ZUR BEREITSTELLUNG VON FEEDBACK IM ZUSAMMENHANG MIT EINER MEDIZINISCHEN UNTERSUCHUNGEN
SYSTÈME, PROCÉDÉ ET PRODUIT PROGRAMME D'ORDINATEUR PERMETTANT DE FOURNIR UN RETOUR D'INFORMATIONS SE RAPPORTANT À UN EXAMEN MÉDICAL

(30) Priority: 11.05.2016 US 201662334477 P
(43) Date of publication of application: 20.03.2019
(73) Proprietor: Tyto Care Ltd., 4250445 Netanya (IL)
(72) Inventor: BYCHKOV, Eyal, 4521464 Hod Hasharon (IL); GILAD-GILOR, David, 4051623 Even Yehuda (IL); DUBIN, Uri, 3440530 Haifa (IL); LACHMANOVICH, Elad, 7176682 Modi'in (IL); DANON, Dov, 2808264 Kiryat Ata (IL); BAUM, Eyal, Tel Aviv 6912105 (IL)
(74) Representative: Goddar, Heinz J.
(86) International application number: PCT/IL2017/050519
(87) International publication number: WO 2017/195203

(56) References cited:
- CN-A- 105 357 501
- US-A- 4 413 630
- US-A- 5 090 042
- US-A1- 2005 149 363
- US-A1- 2008 273 709
- US-A1- 2013 331 664
- US-A1- 2013 331 664
- US-A1- 2016 094 899
- US-B1- 8 734 358

## Description

### FIELD

The invention relates to systems, methods, and computer program products for providing feedback relating to a medical examination.

### BACKGROUND

3M, of Maplewood, Minnesota, markets under the brand name of "Littmann" a series of digital stethoscopes, such as models 3100 and 3200. The 3100 and 3200 display a special symbol on the display (two dashes (--)) in cases the measurement of the heart rate fail. The user manuals of these models indicate several possible causes for failure of determining a heart rate, which include - If the heart rate changes from consistent to inconsistent or if there is excessive ambient noise, patient movement or lung sounds during auscultation. However, inter alia, Littmann's recording of the heart is unrelated to its determination of the heart rate, and the recorded data's quality is not at all determined.

Nonin Medical, Inc. markets a finger pulse oximeter under the name "GO₂" which gives indication of oxygen saturation based on measurements taken at a finger of the patient. A part of the user interface of the GO₂ is referred to as a "Pulse Quality indicator" which displays a strength of a pulse rate signal detected by the device. The "Pulse Quality indicator" provides a visual indicator (number of bars in the display) which indicates the strength of the pulse signal strength.

U.S. patent serial number 8,953,837 entitled "System and Method for Performing an Automatic and Self-Guided Medical Examination" by David Gilad-Gilor of "Tyto Care Ltd.", filed 16 of February, 2012, discloses a method for performing one or more medical examinations of a patient using a diagnostics device, wherein for at least one medical examination of the medical examinations, the method comprising:
a. Providing reference data indicative of a desired spatial disposition of the device with respect to the patient's body for performing the medical examination; operating the device for acquiring navigation enabling data;
b. Determining a spatial disposition of the device with respect to the desired spatial disposition, utilizing the acquired navigation enabling data and the reference data;
c. Calculating a required movement correction from the determined spatial disposition to the desired spatial disposition, for acquiring medical data of the patient in accordance with the at least one medical examination;
d. Providing a user with manoeuvring instructions to navigate the device to the desired spatial disposition in accordance with the calculated route; and
e. Acquiring the medical data upon arrival to the desired spatial disposition.
US 2008/0273709 A1 is directed to systems and methods for tuning, analysis and display of heart sounds from at least one chest location of a patient.

### GENERAL DESCRIPTION

In accordance with a first aspect of the presently disclosed subject matter there is provided a system comprising a processor configured to: obtain physiological data acquired during a non-instantaneous physiological measurement of a patient, wherein the physiological data includes one or more first portions being identified as diagnosis-enabling data and at least one second portion not being identified as diagnosis-enabling data; and display, on the display, a user interface including a progress bar enabling a medical practitioner to navigate to specific points in time within through the obtained physiological data, the user interface including at least one indication, on the progress bar, of a location, of at least one corresponding first portion of the first portions associated with a corresponding point in time, within the obtained physiological data, enabling the user to identify the location.

In some cases, the diagnosing entity is a medical practitioner.

In some cases, the system further comprises a network interface, and wherein the provide includes transmitting, via the network interface, at least the diagnosis-enabling data to a separate device operated by the medical practitioner.
in some cases, the network interface is wireless.

In some cases, the system and the sensor are comprised within a handheld device operated by the user.

In some cases, the sensor is comprised within a handheld device operated by the user and the system is external to the handheld device.

In some cases, the obtain and the analyze are performed in real-time during the medical examination and wherein the processor is further configured to provide the user with an indication of presence of the diagnosis-enabling data if the analysis indicates that the diagnostics enabling data is present within the physiological data.

In some cases, the indication is one or more of the following: (a) a visual indication provided via a user interface of a device operated by the user; (b) a sound indication provided via a speaker of the device operated by the user; (c) a vibration indication provided via a vibrating element within the device operated by the user.

In some cases, the device is a handheld device comprising the sensor.

In some cases, the medical examination is a non-instantaneous physiological measurement conducted over a continuous time period, and wherein the processor is further configured to: determine, at a plurality of points-in-time during the non-instantaneous physiological measurement, a many-valued quality score indicative of a suitability of the currently obtained physiological data for diagnosis by the diagnosing entity; and provide the user with real-time many-valued quality-feedback information which is based on the corresponding determined many-valued quality score.

In some cases, the many-valued quality-feedback information is provided via a user interface of a device operated by the user.

In some cases, the processor is further configured to provide the user with instructions for improving the physiological data acquisition upon the many-valued quality score being lower than a pre-defined threshold.

In some cases, the instructions are instructions for spatially repositioning the sensor with respect to the patient's body.

In some cases, the physiological data is raw data acquired by the sensor.

In some cases, the sensor is an audio sensor and the physiological data is an audio recording.

In some cases, the sensor is a camera and the physiological data is an image or a video recording.

In some cases, the processor is further configured to instruct the user to reacquire the physiological data using the sensor if no presence of diagnosis-enabling data is determined.

In some cases, the processor is configured to selectively provide a success indication for the physiological measurement in response to determining that an accumulative amount of times out of the plurality of different times for which the determined many-valued quality score fulfilled a predetermined criterion exceeded a predetermined amount.

In some cases, the processor is configured to stop the physiological measurement in response to determining that an accumulative amount of times out of the plurality of different times for which the determined many-valued quality score fulfilled a predetermined criterion exceeded a predetermined amount.

In some cases, the physiological data includes (a) first data resulting from a physiological process and (b) second data resulting from additional sources.

In some cases, the additional sources include ambient signal and wherein the analyze includes identifying ambient signal and alerting the user if the ambient signal exceeds a threshold.

In some cases, the processor is further configured to determine a cause of the ambient signal and provide the user with an indication of the determined cause.

In some cases, the analyze includes identifying the first data resulting from the physiological process.

In some cases, the analyze further includes determining presence of diagnosis-enabling data within the first data.

In some cases, the provide includes providing at least part of the obtained physiological data including the diagnosis-enabling data and additional data, and wherein the processor is further configured to provide information indicative of the location of the diagnosis-enabling data within the obtained physiological data.

In some cases, the processor is further configured to instruct the user to reacquire the physiological data using the sensor if no presence of diagnosis-enabling data is determined.

In some cases, the obtain and the analyze are performed in real-time during the medical examination and wherein the processor is further configured to provide the user with an instruction to spatially reposition the sensor with respect to the patient's body in accordance with the medical examination or in accordance with a subsequent medical examination defined by a pre-defined check plan of the patient.

In some cases, the processor is further configured to identify, before obtaining the physiological data, an ambient signal and alert the user if the ambient signal exceeds a threshold.

In some cases, the diagnosing entity is located remotely from the user and from the patient.

In accordance with a second aspect, of the presently disclosed subject matter there is provided a method comprising: obtaining, by a processor, physiological data obtained during a non-instantaneous physiological measurement of a patient, wherein the physiological data includes one or more first portions being identified as diagnosis-enabling data and at least one second portion not being identified as diagnosis-enabling data; and displaying, on a display, by the processor, a user interface including a progress bar enabling a medical practitioner to navigate to specific points in time within the obtained physiological data, the user interface including at least one indication, on the progress bar of a location, of at least one corresponding first portion of the first portions associated with a corresponding point in time within the obtained physiological data, enabling the user to identify the location.

In some cases, the diagnosing entity is a medical practitioner.

In some cases, the provide includes transmitting, via a network interface, at least the diagnosis-enabling data to a separate device operated by the medical practitioner.

In some cases, the network interface is wireless.

In some cases, the processor and the sensor are comprised within a handheld device operated by the user.

In some cases, the sensor is comprised within a handheld device operated by the user and the processor is external to the handheld device.

In some cases, the obtaining and the analyzing are performed in real-time during the medical examination and wherein the method further comprises providing the user with an indication of presence of the diagnosis-enabling data if the analyzing indicates that the diagnostics enabling data is present within the physiological data.

In some cases, the indication is one or more of the following: (a) a visual indication provided via a user interface of a device operated by the user; (b) a sound indication provided via a speaker of the device operated by the user; (c) a vibration indication provided via a vibrating element within the device operated by the user.

In some cases, the device is a handheld device comprising the sensor.

In some cases, the medical examination is a non-instantaneous physiological measurement conducted over a continuous time period, and wherein the method further comprises: determining, by the processor, at a plurality of points-in-time during the non-instantaneous physiological measurement, a many-valued quality score indicative of a suitability of the currently obtained physiological data for diagnosis by the diagnosing entity; and providing the user with real-time many-valued quality-feedback information which is based on the corresponding determined many-valued quality score.

In some cases, the many-valued quality-feedback information is provided via a user interface of a device operated by the user.

In some cases, the method further comprises providing the user with instructions for improving the physiological data acquisition upon the many-valued quality score being lower than a pre-defined threshold.

In some cases, the instructions are instructions for spatially repositioning the sensor with respect to the patient's body.

In some cases, the physiological data is raw data acquired by the sensor.

In some cases, the sensor is an audio sensor and the physiological data is an audio recording.

In some cases, the sensor is a camera and the physiological data is an image or a video recording.

In some cases, the method further comprises instructing the user to reacquire the physiological data using the sensor if no presence of diagnosis-enabling data is determined.

In some cases, the method further comprises selectively providing a success indication for the physiological measurement in response to determining that an accumulative amount of times out of the plurality of different times for which the determined many-valued quality score fulfilled a predetermined criterion exceeded a predetermined amount.

In some cases, the method further comprises stopping the physiological measurement in response to determining that an accumulative amount of times out of the plurality of different times for which the determined many-valued quality score fulfilled a predetermined criterion exceeded a predetermined amount.

In some cases, the physiological data includes (a) first data resulting from a physiological process and (b) second data resulting from additional sources.

In some cases, the additional sources include an ambient signal and wherein the analyze includes identifying the ambient signal and alerting the user if the ambient signal exceeds a threshold.

In some cases, the processor is further configured to determine a cause of the ambient signal and provide the user with an indication of the determined cause.

In some cases, the analyzing includes identifying the first data resulting from the physiological process.

in some cases, the analyzing further includes determining presence of diagnosis-enabling data within the first data.

In some cases, the providing includes providing at least part of the obtained physiological data including the diagnosis-enabling data and additional data, and wherein the method further comprises providing information indicative of the location of the diagnosis-enabling data within the obtained physiological data.

In some cases, the method further comprises instructing the user to reacquire the physiological data using the sensor if no presence of diagnosis-enabling data is determined.

In some cases, the obtaining and the analyzing are performed in real-time during the medical examination and wherein the method further comprises providing the user with an instruction to spatially reposition the sensor with respect to the patient's body in accordance with the medical examination or in accordance with a subsequent medical examination defined by a pre-defined check plan of the patient.

In some cases, the method further comprises identifying, before obtaining the physiological data, an ambient signal and alert the user if the ambient signal exceeds a threshold.

In some cases, the diagnosing entity is located remotely from the user and from the patient.

In accordance with a third aspect of the presently disclosed subject matter there is provided a non-transitory computer readable storage medium having computer readable program code embodied therewith, the computer readable program code, executable by at least one processor to perform a method comprising: obtaining physiological data obtained during a non-instantaneous physiological measurement of a patient, wherein the physiological data includes one or more first portions being identified as diagnosis-enabling data and at least one second portion not being identified as diagnosis-enabling data; and displaying, on a display, a user interface including a progress bar enabling a medical practitioner to navigate to specific points in time within the obtained physiological data, the user interface including at least one indication, on the progress bar, of a location, of at least one corresponding first portion of the first portions associated with a corresponding point in time, within the obtained physiological data, enabling the user to identify the location.

In an example there is provided a system for physiological measurement of a physiological process of a body of a patient, the system comprising: at least one physiological sensor operable to collect multiple times during a physiological measurement physiological data from the body of the patient, the physiological data resulting from: (a) the physiological process and from (b) additional sources; and a processor operable to execute at a plurality of different times during a physiological measurement: identifying parts of the physiological data resulting from the physiological process; based on the physiological data and on results of the identification, determining for the physiological data a many-valued quality score indicative of a suitability of the physiological data for analysis of the physiological process; and providing, by a tangible user interface, many-valued quality-feedback information which is based on the many-valued quality score.

In some cases, the processor is further operable to generate analysis source data for the analysis, based on physiological data collected by the physiological sensor and on at least one of the many-valued quality scores

In some cases, the quality score is different than any value comprised in the analysis source data.

In some cases, the processor is operable to identify the parts of the physiological data which result from the physiological process based on identification of effects of a plurality of different physiological processes on the physiological data.

In some cases, the user interface is operable to present instructions to a user for performing the physiological measurement, wherein the processor determines the instructions based on at least one of the many-valued quality scores.

In some cases, the processor determines at least one or the many-valued quality scores further based on parameters of an analysis procedure selected out of a predefined finite plurality of analysis procedures for analyzing the physiological data.

In some cases, the system is a portable handheld physiological monitoring device.

In some cases, the physiological sensor utilizes for at least one of the measurements an acquisition parameter that is based on at least one of the quality scores.

In some cases, the acquisition parameter is determined further in response to a quality criterion selected for the patient by a medical professional.

In some cases, the acquisition parameter is determined further in response to a medical condition of the patient.

In some cases, the acquisition parameter is determined further in response to quality scores determined with respect to at least one previous physiological measurement which occurred at a previous date.

In some cases, the many-valued quality scores are indicative of a degree in which the patients follows instructions for physical activities.

In some cases, the processor determines the many-valued quality scores based on a selection of a scoring scheme out of a plurality of predefined scoring schemes, wherein each scoring scheme is associated with an analysis process for the physiological process.

In some cases, the processor is configured to compress for the analysis source data different parts of the physiological data based on different many-valued quality scores determined for the different parts.

In some cases, the system further comprises at least one non-physiological sensor, wherein the processor is configured to determine the many-valued quality score for at least one physiological data further based on data collected by the at least one non-physiological sensor.

in some cases, the processor is configured to selectively provide a success indication for the physiological measurement in response to determining that an accumulative amount of times out of the plurality of different times for which the determined many-valued quality score fulfilled a predetermined criterion exceeded a predetermined amount.

In some cases, the processor is configured to stop the physiological measurement in response to determining that an accumulative amount of times out of the plurality of different times for which the determined many-valued quality score fulfilled a predetermined criterion exceeded a predetermined amount.

In some cases, the additional sources include an ambient signal and wherein the analyze includes identifying the ambient signal and alerting the user if the ambient signal exceeds a threshold.

In some cases, the processor is further configured to determine a cause of the ambient signal and provide the user with an indication of the determined cause.

In an example there is provided a computer-implemented method for providing feedback indicative of a suitability of data collected during a physiological measurement for analysis of a physiological process of a body of a patient, the method comprising executing on a processor at a plurality of different times during a physiological measurement the steps of: obtaining physiological data collected from the body of the patient, the physiological data resulting from: (a) the physiological process and from (b) additional sources; identifying parts of the physiological data resulting from the physiological process; based on the physiological data and on results of the identification, determining for the physiological data a many-valued quality score indicative of a suitability of the physiological data for the analysis of the physiological process; and providing, by a tangible user interface, many-valued quality-feedback information which is based on the quality score.

In some cases, the method further comprises generating, based on at least one of the many-valued quality scores and on the physiological data obtained at at least one of the plurality of different times, analysis source data for the analysis of the physiological process.

In some cases, the quality score is different than any value comprised in the analysis source data.

In some cases, the identifying is based on identification of effects of a plurality of different physiological processes on the physiological data.

In some cases, the plurality of different times comprises at least a first time and a second time which is later than the first time, wherein the obtaining of the physiological data at the second time is affected by changes of the physiological measurement by the user as a result from providing by the tangible user interface of the many-valued quality feedback information resulting from many-valued determined for physiological data obtained at the first time.

In some cases, the physiological data is collected by a physiological measurement device; wherein the suitability of the physiological data changes as a result of changes in operating of the physiological measurement device by a user which perceives the quality feedback information presented by the tangible user interface.

In some cases, the method further comprises presenting by the tangible user interface instructions to a user for performing the physiological measurement.

In some cases, the determining of the many-valued quality score is further based on parameters of an analysis procedure selected out of a predefined finite plurality of analysis procedures for analyzing the physiological data.

In some cases, the obtaining, identifying and determining are executed by a portable handheld physiological monitoring device, wherein the obtaining comprises measuring the physiological measurement by at least one physiological sensor of the portable handheld physiological monitoring device.

In some cases, the method further comprises selecting, based on the quality scores, a proper part of the physiological data collected during the physiological measurement, and generating a physiological measurement preview based on the proper part for presenting by a tangible user interface.

In some cases, the method further comprises modifying an acquisition parameter of a physiological sensor which collects at least a part of the measurement data based on at least one of the quality scores.

In some cases, the modifying of the acquisition parameter is executed further in response to a quality criterion selected for the patient by a medical professional.

In some cases, the modifying of the acquisition parameter is executed further in response to a medical condition of the patient.

In some cases, the modifying of the acquisition parameter is executed further in response to quality scores determined with respect to at least one previous physiological measurement which occurred at a previous date.

In some cases, the many-valued quality scores are indicative of a degree in which the patients follows instructions for physical activities.

In some cases, the determining of the many-valued quality score is based on a selection of a scoring scheme out of a plurality of predefined scoring schemes, wherein each scoring scheme is associated with an analysis process for the physiological process.

In some cases, the generating of the analysis source data comprises compressing different parts of the physiological data based on different many-valued quality scores determined for the different parts.

In some cases, the determining of the many-valued quality score for at least, one physiological data is further based on data collected by non-physiological sensor of a physiological measurement system which collected the physiological data.

In some cases, the method further comprises providing a success indication for the physiological measurement in response to determining that an accumulative amount of times out of the plurality of different times for which the determined many-valued quality score fulfilled a predetermined criterion exceeded a predetermined amount.

In some cases, the method further comprises stopping the physiological measurement in response to determining that an accumulative amount of times out of the plurality of different times for which the determined many-valued quality score fulfilled a predetermined criterion exceeded a predetermined amount.

In some cases, the additional sources include an ambient signal and wherein the method further comprises identifying the ambient signal and alerting the user if the ambient signal exceeds a threshold.

In some cases, the method further comprises determining a cause of the ambient signal and providing the user with an indication of the determined cause.

In an example there is provided a non-transitory computer-readable medium for providing feedback indicative of a suitability of data collected during a physiological measurement for analysis of a physiological process of a body of a patient, comprising instructions stored thereon, that when executed on a processor, perform on the processor at a plurality of different times during a physiological measurement the steps of: obtaining physiological data collected from the body of the patient, the physiological data resulting from: (a) the physiological process and from (b) additional sources; identifying parts of the physiological data resulting from the physiological process; based on the physiological data and on results of the identification, determining for the physiological data a many-valued quality score indicative of a suitability of the physiological data for the analysis of the physiological process; and providing, by a tangible user interface, many-valued quality-feedback information which is based on the quality score.

In some cases, the non-transitory computer-readable medium further comprises instructions stored thereon, that when executed on the processor, perform: generating, based on at least one of the many-valued quality scores and on the physiological data obtained at at least one of the plurality of different times, analysis source data for the analysis of the physiological process.

In some cases, the quality score is different than any value comprised in the analysis source data.

In some cases, the identifying is based on identification of effects of a plurality of different physiological processes on the physiological data.

In some cases, the plurality of different times comprises at least a first time and a second time which is later than the first time, wherein the obtaining of the physiological data at the second time is affected by changes of the physiological measurement by the user as a result from providing by the tangible user interface of the many-valued quality feedback information resulting from many-valued determined for physiological data obtained at the first time.

In some cases, the physiological data is collected by a physiological measurement device; wherein the suitability of the physiological data changes as a result of changes in operating of the physiological measurement device by a user which perceives the quality feedback information presented by the tangible user interface.

In some cases, the non-transitory computer-readable medium further comprises instructions stored thereon, that when executed on the processor, perform presenting by the tangible user interface instructions to a user for performing the physiological measurement.

In some cases, the determining of the many-valued quality score is further based on parameters of an analysis procedure selected out of a predefined finite plurality of analysis procedures for analyzing the physiological data.

In some cases, the obtaining, identifying and determining are executed by a portable handheld physiological monitoring device, wherein the obtaining comprises measuring the physiological measurement by at least one physiological sensor of the portable handheld physiological monitoring device.

In some cases, the non-transitory computer-readable medium further comprises instructions stored thereon, that when executed on the processor, perform: selecting, based on the quality scores, a proper part of the physiological data collected during the physiological measurement, and generating a physiological measurement preview based on the proper part for presenting by a tangible user interface.

In some cases, the non-transitory computer-readable medium further comprises instructions stored thereon, that when executed on the processor, perform modifying an acquisition parameter of a physiological sensor which collects at least a part of the measurement data based on at least one of the quality scores.

In some cases, the modifying of the acquisition parameter is executed further in response to a quality criterion selected for the patient by a medical professional.

In some cases, the modifying of the acquisition parameter is executed further in response to a medical condition of the patient.

In some cases, the modifying of the acquisition parameter is executed further in response to quality scores determined with respect to at least one previous physiological measurement which occurred at a previous date.

In some cases, the many-valued quality scores are indicative of a degree in which the patients follows instructions for physical activities.

In some cases, the determining of the many-valued quality score is based on a selection of a scoring scheme out of a plurality of predefined scoring schemes, wherein each scoring scheme is associated with an analysis process for the physiological process.

In some cases, the generating of the analysis source data comprises compressing different parts of the physiological data based on different many-valued quality scores determined for the different parts.

In some cases, the determining of the many-valued quality score for at least one physiological data is further based on data collected by non-physiological sensor of a physiological measurement system which collected the physiological data.

In some cases, the additional sources include an ambient signal and further comprising instructions stored thereon, that when executed on the processor, perform the steps of: identifying the ambient signal and alerting the user if the ambient signal exceeds a threshold.

In some cases, the non-transitory computer-readable medium further comprises instructions stored thereon, that when executed on the processor, perform the step of: determining a cause of the ambient signal and providing the user with an indication of the determined cause.

In an example there is provided a system for physiological measurement of a physiological process of a body of a patient, the system comprising: at least one physiological sensor operable to collect, at a plurality of different times during a physiological measurement, physiological data from the body of the patient, the physiological data resulting at least from the physiological process; and a processor operable to: (a) determine many-valued quality scores for the physiological data collected at the plurality of different times, and (b) to selectively provide a success indication for the physiological measurement in response to determining that an accumulative amount of times, out of the plurality of different times, for which the determined manyvalued quality score fulfilled a predetermined criterion exceeded a predetermined amount.

In an example there is provided a computer-implemented method for providing feedback indicative of a suitability of data collected during a physiological measurement for analysis of a physiological process of a body of a patient, the method comprising executing on a processor: obtaining physiological data collected from the body of the patient at. a plurality of different times during a physiological measurement, the physiological data resulting at least from the physiological process; determining many-valued quality scores for the physiological data collected at the plurality of different times: and selectively providing a success indication for the physiological measurement in response to determining that an accumulative amount of times, out of the plurality of different times, for which the determined many-valued quality score fulfilled a predetermined criterion exceeded a predetermined amount.

In an example there is provided a non-transitory computer-readable medium for providing feedback indicative of a suitability of data collected during a physiological measurement for analysis of a physiological process of a body of a patient, comprising instructions stored thereon, that when executed on a processor, perform on the processor at a plurality of different times during a physiological measurement the steps of: obtaining physiological data collected from the body of the patient at a plurality of different times during a physiological measurement, the physiological data resulting at least from the physiological process; determining many-valued quality scores for the physiological data collected at the plurality of different times; and selectively providing a success indication for the physiological measurement in response to determining that an accumulative amount of times, out of the plurality of different times, for which the determined many-valued quality score fulfilled a predetermined criterion exceeded a predetermined amount.

In an example there is provided a system comprising a processor and a display, wherein the processor is configured to: obtain physiological data obtained during a non- instantaneous physiological measurement of a patient, wherein the physiological data includes one or more first portions being identified as diagnosis-enabling data and at least one second portion not being identified as diagnosis-enabling data; and display, on the display, a user interface enabling a medical practitioner to navigate through the physiological data, the user interface including at least one indication of a location, of at least one corresponding first portion of the first portions, within the obtained physiological data, enabling the user to identify the location.

In some cases, the physiological data is an audio or video recording and the indication includes a first marking, on a video or audio progress bar displayed on the user interface and associated with the physiological data, of a start location of the at least one corresponding first portion.

In some cases, the indication includes a second marking, on the video or audio progress bar, of an end location of the at least one corresponding first portion.

In some cases, the indication includes a graph representing a plurality of many-valued quality scores, each calculated for a corresponding point-in-time during the non-instantaneous physiological measurement, and each indicative of a suitability of the physiological data in the corresponding point-in-time for diagnosis by a medical practitioner.

In some cases, the length of the video or audio recording is at least ten seconds.

In some cases, the physiological measurement being conducted by a user using a sensor comprised within a handheld diagnosis-device, wherein the user is not the medical practitioner.

In some cases, the physiological data is obtained during the physiological measurement conducted at a first geographical location and transmitted to a second geographical location of the medical practitioner, the second geographical location being remote from the first geographical location.

In some cases, the processor is further configured to: receive, from the medical practitioner, an indication of an area-of-interest within the physiological data; and send the physiological data and the indication of the area-of-interest to a remote workstation operated by a second medical practitioner, thereby enabling the remote workstation to present the physiological data and the indication of the area-of-interest to the second medical practitioner for analysis purposes.

In some cases, the processor is further configured to display, on the display, a navigation User Interface (UI) element, wherein upon activation of the navigation UI element, the system automatically navigates to a next or a previous first position of the first positions, thereby enabling skipping the second portions.

In some cases, the processor is further configured to: receive, from the medical practitioner, an indication of an area-of-interest within the physiological data; and store the indication in an Electronic Health Record (EHR) associated with the patient.

In an example there is provided a method comprising: obtaining, by a processor, physiological data obtained during a nori-instantaneous physiological measurement of a patient, wherein the physiological data includes one or more first portions being identified as diagnosis-enabling data and at least one second portion not being identified as diagnosis-enabling data; and displaying, on a display, by the processor, a user interface enabling a medical practitioner to navigate through the physiological data, the user interface including at least one indication of a location, of at least one corresponding first portion of the first portions, within the obtained physiological data, enabling the user to identify the location.

in some cases, the physiological data is an audio or video recording and the indication includes a first marking, on a video or audio progress bar displayed on the user interface and associated with the physiological data, of a start location of the at least one corresponding first portion.

In some cases, the indication includes a second marking, on the video or audio progress bar, of an end location of the at least one corresponding first portion.

In some cases, the indication includes a graph representing a plurality of many-valued quality scores, each calculated fora corresponding point-in-time during the non-instantaneous physiological measurement, and each indicative of a suitability of the physiological data in the corresponding point-in-time for diagnosis by a medical practitioner.

in some cases, the length of the video or audio recording is at least ten seconds.

In some cases, the physiological measurement being conducted by a user using a sensor comprised within a handheld diagnosis-device, wherein the user is not the medical practitioner.

in some cases, the physiological data is obtained during the physiological measurement, conducted at a first geographical location and transmitted to a second geographical location of the medical practitioner, the second geographical location being remote from the first geographical location.

In some cases, the method further comprises: receiving, by the processor, from the medical practitioner, an indication of an area-of-interest within the physiological data; and sending, by the processor, the physiological data and the indication of the area-of-interest to a remote workstation operated by a second medical practitioner, thereby enabling the remote workstation to present the physiological data and the indication of the area-of-interest to the second medical practitioner for analysis purposes.

In some cases, the method further comprises displaying, on the display, a navigation User Interface (UI) element, wherein upon activation of the navigation UI element, the processor automatically navigates to a next or a previous first position of the first positions, thereby enabling skipping the second portions.

In some cases, the method further comprises: receiving, by the processor, from the medical practitioner, an indication of an area-of-interest within the physiological data; and storing, by the processor, the indication in an Electronic Health Record (EHR) associated with the patient.

In an example there is provided a non-transitory computer readable storage medium having computer readable program code embodied therewith, the computer readable program code, executable by at least, one processor to perform a method comprising: obtaining physiological data obtained during a non-instantaneous physiological measurement of a patient, wherein the physiological data includes one or more first portions being identified as diagnosis-enabling data and at least one second portion not being identified as diagnosis-enabling data; and displaying, on a display, a user interface enabling a medical practitioner to navigate through the physiological data, the user interface including at least one indication of a location, of at least one corresponding first portion of the first portions, within the obtained physiological data, enabling the user to identify the location.

in an example there is provided a system comprising a processor and a display, wherein the processor is configured to: obtain, for each patient of a plurality of patients, one or more files associated with the patient, each comprising physiological data acquired during a corresponding non-instantaneous physiological measurement for analysis of a physiological process of the corresponding patient's body, and each file having a quality score indicative of a suitability of the physiological data comprised therein for diagnosis by a medical practitioner; and display, on the display, (a) a list of the patients, and (b) for at least one of the patients at least one indication of a patient medical examination quality score.

In some cases, the patient medical examination quality scores is a maximal quality score of the files associated with the corresponding patient and wherein the list is ordered at least by the maximal quality scores.

In some cases, the processor is further configured to display, upon selection of a given patient of the patients, on the display, a second list of the files associated with the given patient, and, for each of the files, the quality score thereof.

In some cases, the quality score of each file of the files is a maximal score out of a plurality of many-valued quality scores calculated for corresponding points- in-time during the corresponding non-instantaneous physiological measurement, each of the many-valued quality scores being indicative of a suitability of the physiological data in the corresponding point-in-time for diagnosis by a medical practitioner.

In some cases, the physiological data includes one or more first portions being identified as diagnosis-enabling data and at least one second portion not being identified as diagnosis-enabling data, and wherein the processor is further configured to display, upon selection of a given file of the files displayed on the display, a user interface enabling a medical practitioner to navigate through the physiological data, the user interface including at least one indication of a location, of at least one corresponding first portion being identified as diagnosis-enabling data, within the obtained physiological data, enabling the user to identify the location.

In some cases, the physiological data is an audio or video recording and the indication includes a first marking, on a video or audio progress bar displayed on the user interface and associated with the physiological data, of a start location of the at least one corresponding first portion.

In some cases, the indication includes a second marking, on the video or audio progress bar, of an end location of the at least one corresponding first portion.

In some cases, the indication includes a graph representing a plurality of many-valued quality scores, each calculated for a corresponding point-in-time during the non-instantaneous physiological measurement, and each indicative of a suitability of the physiological data in the corresponding point-in-time for diagnosis by a medical practitioner.

In some cases, the length of the video or audio recording is at least ten seconds.

In some cases, the physiological measurement being conducted by a user using a sensor comprised within a handheld diagnosis-device, wherein the user is not the medical practitioner.

In some cases, the physiological data is obtained during the physiological measurement conducted at a first geographical location and transmitted to a second geographical location of the medical practitioner, the second geographical location being remote from the first geographical location.

in some cases, the processor is further configured to: receive, from the medical practitioner, an indication of an area-of-interest within the physiological data; and send the physiological data and the indication of the area-of-interest to a remote workstation operated by a second medical practitioner, thereby enabling the remote workstation to present the physiological data and the indication of the area-of-interest to the second medical practitioner for analysis purposes.

In some cases, the list is ordered in a descending order of the maximal quality scores.

In an example there is provided a method comprising: obtaining, by a processor, for each patient of a plurality of patients, one or more files associated with the patient, each comprising physiological data acquired during a corresponding non-instantaneous physiological measurement for analysis of a physiological process of the corresponding patient's body, and each file having a quality score indicative of a suitability of the physiological data comprised therein for diagnosis by a medical practitioner; and displaying, on the display, by a processor, (a) a list of the patients, and (b) for at least one of the patients at least one indication of a patient medical examination quality score.

In some cases, the patient medical examination quality scores is a maximal quality score of the files associated with the corresponding patient and wherein the list is ordered at least by the maximal quality scores.

In some cases, the method further comprises displaying, upon selection of a given patient of the patients, on the display, a second list of the files associated with the given patient, and, for each of the files, the quality score thereof.

In some cases, the quality score of each file of the files is a maximal score out of a plurality of many-valued quality scores calculated for corresponding points- in-time during the corresponding non-instantaneous physiological measurement, each of the many-valued quality scores being indicative of a suitability of the physiological data in the corresponding point-in-time for diagnosis by a medical practitioner.

In some cases, the physiological data includes one or more first portions being identified as diagnosis-enabling data and at least one second portion not being identified as diagnosis-enabling data, and wherein the method further comprises displaying, by the processor, upon selection of a given file of the files displayed on the display, a user interface enabling a medical practitioner to navigate through the physiological data, the user interface including at least one indication of a location, of at least one corresponding first portion being identified as diagnosis-enabling data, within the obtained physiological data, enabling the user to identify the location.

In some cases, the physiological data is an audio or video recording and the indication includes a first marking, on a video or audio progress bar displayed on the user interface and associated with the physiological data, of a start location of the at least one corresponding first portion.

In some cases, the indication includes a second marking, on the video or audio progress bar, of an end location of the at least one corresponding first portion.

In some cases, the indication includes a graph representing a plurality of many-valued quality scores, each calculated for a corresponding point-in-time during the non-instantaneous physiological measurement, and each indicative of a suitability of the physiological data in the corresponding point-in-time for diagnosis by a medical practitioner.

In some cases, the length of the video or audio recording is at least ten seconds.

In some cases, the physiological measurement being conducted by a user using a sensor comprised within a handheld diagnosis-device, wherein the user is not the medical practitioner.

In some cases, the physiological data is obtained during the physiological measurement conducted at a first geographical location and transmitted to a second geographical location of the medical practitioner, the second geographical location being remote from the first geographical location.

In some cases, the method further comprises: receiving, by the processor, from the medical practitioner, an indication of an area-of-interest within the physiological data; and sending, by the processor, the physiological data and the indication of the area-of-interest to a remote workstation operated by a second medical practitioner, thereby enabling the remote workstation to present the physiological data and the indication of the area-of-interest to the second medical practitioner for analysis purposes.

In some cases, the list is ordered in a descending order of the maximal quality scores.

In an example there is provided a non-transitory computer readable storage medium having computer readable program code embodied therewith, the computer readable program code, executable by at least one processor to perform a method comprising: obtaining, for each patient of a plurality of patients, one or more files associated with the patient, each comprising physiological data acquired during a corresponding non-instantaneous physiological measurement for analysis of a physiological process of the corresponding patient's body, and each file having a quality score indicative of a suitability of the physiological data comprised therein for diagnosis by a medical practitioner; and displaying, on the display, (a) a list of the patients, and (b) for at least one of the patients at least one indication of a patient medical examination quality score.

### BRIEF DESCRIPTION OF THE DRAWINGS

In order to understand the invention and to see how it may be carried out in practice, embodiments will now be described, by way of non-limiting example only, with reference to the accompanying drawings, in which:
Figs. 1 and 2 are functional block diagrams illustrating examples of systems, in accordance with the presently disclosed subject matter;
Figs. 3A, 3C, 4 and 6 are flow charts illustrating examples of a method for providing feedback indicative of a suitability of data collected during a physiological measurement for analysis of a physiological process of a body of a patient, in accordance with the presently disclosed subject matter;
Fig. 3B is a flow chart illustrating an example of the method of Fig. 3A adapted for preparing analysis source data for analysis of a physiological process of a body of a patient, in accordance with the presently disclosed subject matter;
Fig. 5 illustrates optional ways of implementing a stage of the method of Figs. 3, 4 and 6, in accordance with examples of the presently disclose subject matter;
Fig. 7 illustrates an optional stage of the method of Figs. 3, 4 and 6, according to examples of the presently disclosed subject matter;
Fig. 8 is a flow chart illustrating an example of a method for providing feedback indicative of a suitability of data collected during a pulmonary auscultation for pulmonary analysis of a patient, in accordance with the presently disclosed subject matter;
Fig. 9 is a flow chart illustrating an example of a method for a user of a system, in accordance with the presently disclosed subject matter;
Fig. 10 is a flowchart illustrating an example of a method for providing feedback indicative of presence/absence of diagnosis-enabling data within physiological data collected from a body of a patient, in accordance with the presently disclosed subject matter;
Fig. 11 is an illustration of a user interface shown on a display of a medical practitioner system and enabling navigation to Points of Interest (POIs) within physiological data obtained during a non-instantaneous physiological measurement, in accordance with the presently disclosed subject matter;
Fig. 12 is a functional block diagram illustrating an exemplary medical practitioner system, in accordance with the presently disclosed subject matter;
Fig. 13 is a flowchart illustrating one example of a sequence of operations carried out for enabling navigation to Points/Areas of Interest (POls) within physiological data obtained during a non-instantaneous physiological measurement, in accordance with the presently disclosed subject matter;
Fig. 14 a flowchart illustrating one example of a sequence of operations carried out for providing a second medical practitioner with physiological data and an indication of areas-of-interest for consideration, in accordance with the presently disclosed subject matter;
Fig. 15 is an illustration of another user interface shown on a display of a medical practitioner system and enabling a medical practitioner to manage virtual visits of a plurality of patients, in accordance with the presently disclosed subject matter; and
Fig. 16 a flowchart illustrating one example of a sequence of operations carried out for enabling a medical practitioner to manage virtual visits of a plurality of patients, in accordance with the presently disclosed subject matter.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

In the drawings and descriptions set forth, identical reference numerals indicate those components that are common to different embodiments or configurations.

Unless specifically stated otherwise, as apparent from the following discussions, it is appreciated that throughout the specification discussions utilizing terms such as "processing", "determining", "generating", or the like, include action and/or processes of a computer that manipulate and/or transform data into other data, said data represented as physical quantities, e.g. such as electronic quantities, and/or said data representing the physical objects. The terms "computer", "processor", and "controller" should be expansively construed to cover any kind of electronic device with data processing capabilities, including, by way of non-limiting example, a personal computer, a server, a computing system, a communication device, a processor (e.g. digital signal processor (DSP), a microcontroller, a field programmable gate array (FPGA), an application specific integrated circuit (ASIC), etc.), any other electronic computing device, and or any combination thereof.

The operations in accordance with the teachings herein may be performed by a computer specially constructed for the desired purposes or by a general-purpose computer specially configured for the desired purpose by a computer program stored in a computer readable storage medium.

As used herein, the phrase "for example," "such as", "for instance" and variants thereof describe non-limiting embodiments of the presently disclosed subject matter. Reference in the specification to "one case", "some cases", "other cases" or variants thereof means that a particular feature, structure or characteristic described in connection with the embodiment(s) is included in at least one embodiment of the presently disclosed subject matter. Thus, the appearance of the phrase "one case", "some cases", "other cases" or variants thereof does not necessarily refer to the same embodiment(s).

It is appreciated that certain features of the presently disclosed subject matter, which are, for clarity, described in the context of separate embodiments, may also be provided in combination in a single embodiment. Conversely, various features of the presently disclosed subject matter, which are, for brevity, described in the context of a single embodiment, may also be provided separately or in any suitable sub-combination.

In embodiments of the presently disclosed subject matter one or more stages illustrated in the figures may be executed in a different order and/or one or more groups of stages may be executed simultaneously and vice versa. The figures illustrate a general schematic of the system architecture in accordance with an embodiment of the presently disclosed subject matter. Each module in the figures can be made up of any combination of software, hardware and/or firmware that performs the functions as defined and explained herein. The modules in the figures may be centralized in one location or dispersed over more than one location.

Any reference in the specification to a method should be applied mutatis mutandis to a system capable of executing the method and should be applied mutatis mutandis to a non-transitory computer readable medium that stores instructions that once executed by a computer result in the execution of the method.

Any reference in the specification to a system should be applied mutatis mutandis to a method that may be executed by the system and should be applied mutatis mutandis to a non-transitory computer readable medium that stores instructions that may be executed by the system.

Any reference in the specification to a non-transitory computer readable medium should be applied mutatis mutandis to a system capable of executing the instructions stored in the non-transitory computer readable medium and should be applied mutatis mutandis to method that may be executed by a computer that reads the instructions stored in the non-transitory computer readable medium.

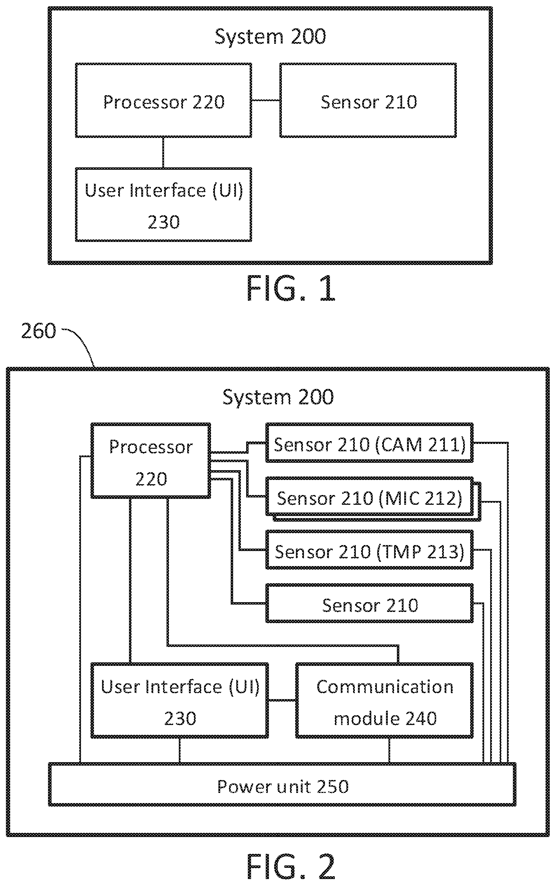

Fig. 1 is a functional block diagram illustrating an example of a system 200, in accordance with the presently disclosed subject matter.

While not necessarily so, system 200 may be a portable unit, a mobile unit, a handheld unit, etc. System 200 may be a user activated mobile device, which is designed to be operated by a user without medical training (also referred to herein as "a non-medical practitioner"). Optionally, system 200 may be a smartphone, or another computer which optionally includes one or more different types of sensors. Optionally, system 200 may be a dedicated portable handheld device which includes one or more sensors and a processor, such as the handheld medical devices produced by Tytocare LTD. Of Netanya, Israel. System 200 may optionally be a handheld physiological monitoring device, or any device capable of acquiring physiological data during a medical examination of a patient, or any device capable of obtaining, e.g. via a wired/wireless communication channel, physiological data acquired (by the device or optionally by another device, other than system 200) during a medical examination of a patient.

The term "Physiological measurement" which is well accepted in the art, should be construed in a non-limiting way to include a process of monitoring, over a span of time, a physiological process that optionally changes in time (heart, breathing, lungs, blood saturation, temperature, tympanic membrane view, body part observation (observation meaning a non-instantaneous view of the body part), tonsil observation). Within the scope of the present disclosure, the term "physiological measurement" does not refer to instantaneous measurement, but rather to measurements which extend over a longer period of time (e.g. more than one second, could also be minutes and beyond). The physiological measurement can be related to one or more medical examinations of the patient (e.g. a medical examination of the patient's lungs/heart/throat/skin or any other medical examination of the patient).

That said, it is to be noted that the term "physiological data" should be construed in a non-limiting way to include data obtained non-instantaneously or instantaneously, during a medical examination of the patient. Some examples of physiological data include an image or a video of the patient or relevant body part/s thereof, a blood sample of the patient's blood, or any other parameter representing a physiological characteristic of a patient. The physiological data is collected or obtained by system 200 (e.g. utilizing at least one physiological sensor 210), and may also be referred to as "physiological reading". The patient may be any person (or animal) whose physiological parameters are to be measured, whether if for medical use or for any other use (e.g. estimating effectivity of physical training, and so on). It is to be noted that the physiological data can be a raw reading obtained utilizing at least one physiological sensor 210.

System 200 comprises a processor 220 capable of processing and/or analyzing physiological data. The physiological data can be acquired from the patient's body by at least one physiological sensor 210. The at least one physiological sensor 210 can optionally be comprised within the system 200. In other cases, the at least one physiological sensor 210 can be external to the system 200, and system 200 can obtain the physiological data obtained by the at least one physiological sensor 210 via a wired/wireless communication channel.

In some cases, the processor 220 can be configured to obtain physiological data (acquired by the at least one physiological sensor 210 during a medical examination of the patient's body) and to analyze it to determine presence of diagnosis-enabling data therein. Diagnosis-enabling data is regarded as data that enables a diagnosing entity, such as a medical practitioner (e.g. a physician, a technician) or a computerized system configured to diagnose medical conditions based on physiological data, to perform a diagnosis of a medical condition of the patient. It is to be noted that in order for a diagnosing entity to be able to diagnose based on the diagnosis-enabling data, the diagnosis-enabling data is required to be of a certain minimal quality, that enables such diagnosis. If the analysis shows that diagnosis-enabling data exists within the obtained physiological data, the processor 220 can be further configured to provide at least the diagnosis-enabling data to the diagnosing entity, thereby enabling the diagnosing entity to diagnose the medical condition of the patient.

It is to be noted that the user operating the system 200 is not necessarily a medical practitioner, or is a medical practitioner that is not authorized to, or capable of, diagnosing physiological data (e.g. a nurse), and at least in some cases, the user is not a medical practitioner authorized to, or capable of, diagnosing the physiological data. The user operating the system 200 can be the patient from which the physiological data is obtained or another non-medical practitioner such as a family member of the patient, or a medical practitioner not authorized to, or capable of, diagnosing physiological data (e.g. a nurse). Such user (a non-medical practitioner, or a medical practitioner not authorized to, or capable of, diagnosing physiological data), in many cases, does not have the ability to determine if the physiological data obtained from the patient's body includes diagnosis-enabling data. In many cases, the physiological data is obtained without any assistance, and in more specific cases, without real-time assistance, from a medical practitioner authorized to, or capable of, diagnosing physiological data. Therefore, there is a need for the system 200 to provide the user with feedback that will enable such user to operate the system 200 in a manner that will result in diagnosis-enabling data being sent to the medical practitioner (that can be located remotely from the patient) for analysis (optionally at a later time, after the physiological data obtainment is complete). Otherwise, the medical examination of the patient (during which the physiological data is collected) will have to be repeated in order to obtain new or additional physiological data from the patient's body. This naturally requires the availability of the patient, and optionally, if the patient is not operating the sensor himself, also of a user operating the system 200. In addition to the need to repeat the medical examination, in case the physiological data does not comprise diagnosis-enabling data, the diagnosing entity's resources are wasted, as it will attempt to diagnose a medical condition of the patient based on data that does not comprise diagnosis-enabling data, and therefor - it will fail, or provide a poor or an erroneous diagnosis.

In some cases, the processor 220 can be further configured to provide a user operating the system 200 (e.g. the patient or another non-medical practitioner such as a family member of the patient), e.g. using a user interface 230, with an indication of presence/absence of diagnosis-enabling data within the obtained physiological data, if and when the analysis shows that the physiological data comprises diagnosis-enabling data. In some cases, the indication can be a V mark if diagnosis-enabling data is present and an X mark of diagnosis-enabling data is absent within the obtained physiological data.

In some cases, the physiological data is obtained and analyzed in real-time (e.g. immediately, or substantially immediately, after the physiological data is acquired by the at least one physiological sensor 210). In some cases, the indication of presence of diagnosis-enabling data within the obtained physiological data is also provided in real-time (e.g. immediately, or substantially immediately, after determining that diagnosis-enabling data exists within the obtained physiological data), thereby enabling the user operating the system 200 to determine when to stop performance of the physiological examination of the patient. It is to be noted that in some cases the indication of presence of diagnosis-enabling data within the obtained physiological data can be provided at a certain point-in-time after the processor 220 determines that diagnosis-enabling data exists within the obtained physiological data (e.g. up to a few seconds or minutes later).

In some cases, the processor 220 can be further configured to utilize the physiological data for generating many-valued quality-feedback information, which is provided to a user, optionally in real-time (e.g. immediately, or substantially immediately, after generating the many-valued quality-feedback information), e.g. using a user interface 230. Optionally, processor 220 may also be configured to process the physiological data for generating analysis source data for an analysis of a physiological process of a body of a patient. It is to be noted that the analysis source data includes at least diagnosis-enabling data.

Processor 220 can optionally generate (if it is configured to do so) the analysis source data from the physiological data in view of one or more of the many-valued quality scores which are assigned by processor 220 to physiological data collected by sensor 210 at different times. These quality scores are indicative of a suitability of the physiological data for the analysis of the specific physiological process - and are based on identification of parts of the physiological data which result from the physiological process (as some of the physiological data results from other sources such as noise, ambient conditions, or other physiological process which are also sampled by the physiological sensor).

The "suitability of the physiological data for the analysis of the specific physiological process" should be construed in a non-limiting way to include a degree in which the physiological data - or a processed version of it - can be used by (i.e. is useful for) known systems or processes for analysis of the specific physiological process. The intended analysis may be executed by a computer or another machine and/or by one or more persons (e.g. a medical practitioner such as a physician, a technician, a nurse, etc.).

The analysis of the physiological process may include, for example, any one or more of the following: determining the condition or the nature of the physiological process, prognosis of a medical condition associated with the physiological process, diagnosis of the physiological process or associated physiological process, classification of the physiological process, and so on. Some examples of physiological data which are less or more useful for analysis of different physiological processes are provided below.

The physiological process may involve one or more organs (e.g. breathing, heartbeats, blinking, etc.). Other examples of physiological processes which may be analyzed using the physiological data collected by system 200 include: body temperature of one or more organs, electrocardiogram (ECG) measurements, audio signals (e.g. of the heart operations or of the lungs), ultrasound signals (e.g. of the heart, of the intestines, etc.), body tissue electrical resistance, hardness of body tissues, and so on.

The physiological process may be measured using audio capturing physiological sensor (e.g. a microphone). For example, system 200 may be used for auscultation of the heart. The physiological sensor - a microphone, in this example-may sample not only sounds of the heart, but also other sounds such as sounds arriving from the lungs (resulting from the physiological process of breathing). In such a case, simple tests (e.g. measuring the volume level of the microphone signal) are insufficient to determine the suitability of the collected sound signal for analysis of the heart - because the origin of the sound cannot be assessed in such simple tests.

The physiological process may be measured using image/video capturing physiological sensor (e.g. a camera). For example, physiological sensor 210 may be a camera which is intended to monitor breathing of the patient by movement of the chest. The camera may also collect movements of the chest having different origins, such as body movement of the patient, muscular movements, and so on. The suitability of the video collected by the camera for analyzing the breathing of the patient does not depend only on the magnitude of movement of the chest as reflected by the video - because this movement can also be unrelated to the breathing.

Therefore, processor 220 can be configured to identify the parts of the physiological data which result from the physiological process (e.g. the heartbeats and the breathing of the previous examples), and to determine for the collected physiological data a quality score which is indicative of the suitability of the physiological data for the analysis of the physiological process. Processor 220 can be configured to determine the quality score based on the physiological data and on results of the identification. Therefore, the quality score assigned to the collected data is not simply based on the collected signal in its entirety, but also on differentiation between parts of the collected physiological data which are identified to result from the physiological process and other parts of the physiological data. As discussed below, system 200 can use the collected physiological data and the quality score in different ways.

For example, the quality score may be provided to a user of system 200 as a feedback (e.g. using a user interface, UI), optionally in real-time (or near-real time, e.g. with up to one second delay or a couple of seconds delay) during performance of the medical examination, so that the user can adapt and therefore improve the collection of physiological data. System 200 may optionally provide instructions regarding ways of changing the measurement process in order to improve the quality of physiological data collection for the specific physiological process. Optionally, processor 220 may use the quality score to determine that sufficient data was collected (i.e. that the obtained physiological data comprises diagnosis-enabling data). Based on the results of such a decision, system 200 may indicate to the user an end of measurement - or of a part of the measurement process. For example, the system may instruct the user to move it to another location on the body to continue the measurement.

The feedback and/or instructions provided by system 200 may optionally include information regarding possible causes for interference or degradations of the collected physiological data. The feedback and/or instructions provided by system 200 may optionally include information regarding actions which may be taken by a user of the system in order to overcome such interferences or possible causes for degradations of the collected physiological data.

In another example, processor 220 may use the quality score in order to process the collected physiological data, and to generate analysis source data which is more suitable for analysis of the physiological process. For example, the analysis source data may omit parts of the collected physiological data which are less suitable for analysis of the specific physiological process (e.g. heart beating, breathing). As another example, the analysis source data may be processed to reduce the relative effect of other contributors to the collected physiological data, etc. It is to be noted that the analysis source data can be data that enables diagnosis of the physiological process (diagnosis-enabling data).

While not necessarily so, processor 220 may also be configured to automatically analyze the physiological process, based on the analysis source data. The results of such an analysis may be displayed to the user, stored in a memory of system 200, and/or transmitted to a remote system (e.g. a server, a doctor station, etc.).

While not necessarily so, system 200 may be designed to be operated by a non-medical practitioner, or a non-professional operator (or a semi-professional operator), such as the patient herself, a family member of the patient, or another operator which is not specifically trained to undertake the respective one or more physiological measurements offered by the system.

While not necessarily so, system 200 may be a standalone unit which includes a rigid casing 260 in which processor 220, power assembly 250 and other components are included. The standalone unit (if so implemented) may also include elements residing (partly or wholly) outside the casing, such as cables, electrodes, connectors, etc.

Rigid casing 260 may encompass one or more physiological sensors 210, but one or more physiological sensors 210 may also be external to casing 260. It is noted that optionally, system 200 may utilize one or more external physiological sensors 210 which are not physically connected to processor 220. Such a physiological sensor 210 may even be a part of another system. For example, the microphone of a smartphone with which system 200 have established a communication channel may serve as a physiological sensor 210. However, optionally all of the one or more sensors 210 utilized by system 200 are parts of a single unit.

System 200 can include one or more physiological sensors 210, each of which is operable to collect physiological data from a patient. The physiological data may be collected directly from the body of the patient, but may also be processed during the collection process. For example, an audio signal may pass through a low-pass, band-pass, or high-pass filters. In another example, a photo or video signal may be corrected for lighting or contrast level.

It is noted that if system 200 includes more than one physiological sensor 210, the plurality of physiological sensors 210 may be all of the same kinds (e.g. two microphones), or of two or more kinds (e.g. a microphone, a temperature sensor and a camera). Different physiological sensors 210 may operate together in unison for collecting data relating to a single physiological process (e.g. collecting both video and temperature measurements relating to the ear canal, or measuring sound signals by two microphones), but this is not necessarily so.

It is noted that the physiological data may be collected by physiological sensor 210 from different parts of the body of the patient - depending on the type of physiological sensor (e.g. camera, microphone, EEG, thermometer, etc.), on the purpose of physiological examination (e.g. diagnosis of a medical condition, auscultating the heart, auscultating the lungs, monitoring a development of a skin mole over time, etc.), etc. The examined body location may be a superficial location on the body of the patient (i.e. on the skin, or otherwise on one or more external features of the body, such as the eyes, the fingernails, etc.) or internal (e.g. heart, lungs, bladder, etc.).

Physiological sensor 210 may optionally be located completely outside the body of the patient when acquiring the physiological data. In other situations, or implementations, parts or whole of physiological sensor 210 may optionally enter the body of the patient (e.g. a needle penetrating the skin and/or a blood vessel, a sensor entering a body orifice such as the ear or the mouth, and so on).

While not necessarily so, in some cases, at least one physiological sensor 210 of system 200 is used at different times during a single physiological measurement to collect physiological data from the body of the patient. The physiological data may be collected by one or more sensors. Collection by a plurality of sensors - if implemented - may be executed in unison (e.g. a plurality of EEG electrodes may provide information for cardiogram), and may also be executed by uncoordinated sensors (e.g. measuring both temperature and video data inside the ear canal).

The physiological data collected can result from the physiological process (e.g. heartbeats, lungs breathing in and out), but also from additional sources. For example, the additional sources may be other physiological processes (e.g. blood flowing in vessels, digestion, breathing, heartbeats, etc.), ambient signals (e.g. ambient sounds, lights, temperature, etc.), and so on. Ambient sounds can be sounds that originate from one or more of the following: people talking, wind blowing, car traffic noise, air-conditioning noise, dog barks, noises resulting from the operation of system 200 (e.g. friction of the system 200 over the patient's body or clothes), or any other noise not originating from the physiological process.

Since the physiological data collected by system 200 is intended to be used in analysis of the physiological process (e.g. for diagnosis of a medical condition of the patient), the data resulting from the physiological process is, in most cases, more important than the data resulting from the additional sources. The relative parts of the data resulting from the physiological process and of the data resulting from other sources in the collected physiological data may differ - and at some situations the effect of the additional sources on the collected data signal may be larger than that of the physiological process. This situation is dealt with by system 200, and especially by processor 220, as discussed below.

When analyzing a physiological process, the physiological data collected by the one or more physiological sensors 210 is communicated to processor 200 during the collection of the physiological data (i.e. some of the physiological data is transferred to processor 220 - and it is processed by processor 220 - before the last of the physiological data is collected). In such cases, t a plurality of different times during the physiological measurement (i.e. in real time or near real time, in parallel with the collection), processor 220 can be configured to perform the following:
a. identify parts of the physiological data resulting from the physiological process;
b. Based on the physiological data and on results of the identification, determine for the physiological data a many-valued quality score indicative of a suitability of the physiological data for the analysis of the physiological process;
c. Following that, provide, by a tangible user interface, many-valued quality-feedback information which is based on the many-valued quality score.

After the end of the measurement (or, optionally, before the measurement ends), processor 220 may optionally generate the analysis source data based on at least one of the many-valued quality scores, and on the physiological data obtained at at least one of the plurality of different times. In the following paragraphs, more details will be provided with respect to any one of those operations (discussed in the previous paragraph and in this one) which processor 220 can be operable and configured to execute.

As mentioned above, processor 220 identifies parts of the physiological data which result from the physiological process. This may be done by different types of algorithms and/or electric circuits (including digital signal processing and/or analog signal processing). It is noted that the identification of the parts of the physiological data which result from the physiological process may include identifying the physiological process (e.g. heartbeats) but may additionally, or alternatively, include (in addition or instead) identifying other parts of the physiological data (e.g. other physiological processes such as breathing, ambient signals (e.g. ambient noise, etc.), etc.). The identification of other parts of the physiological data (which do not result from the physiological process) may be used to eliminate such part and/or to assist in identifying the parts of the physiological data which result from the physiological process. It is to be noted that identification of other parts of the physiological data (which do not result from the physiological process) may additionally, or alternatively, be used for identifying a source of interference (e.g. the source of the other parts of the physiological data) and providing the user with instructions for reducing, or eliminating, such interference, as further detailed herein.

It is noted that the term "part" with respect to parts of the physiological data may refer to different types of parts in different implementations of the presently disclosed subject matter. For example, different parts of the physiological data may be distinguished from one another using any combination of any one or more of the following - by time, by frequency, by temporal and/or frequency pattern, etc.

Processor 220 can also be operable to determine at different times during the physiological measurement, based on the physiological data and on results of the identification, a many-valued quality score indicative of a suitability of the physiological data for the analysis of the physiological process.

The term "many-valued score" (e.g. "many-valued quality score") means that the value of the score has more than two options (i.e. it is not a constant and not a binary value). A many-valued score describes more than two states (e.g. 1 or 0, pass or fail, etc.). It is noted that the many-valued quality score may be discrete or continuous, and may have a predefined set of optional values, but this is not necessarily so. For example, the many-valued quality score determined by processor 220 may be an integer between 1 and 5, an integer between 1 and 10, a decimal number between 0 and 100, one of several descriptive words/phrases (e.g. "good", "medium", "low", and "fail"), and so on.

The "Results of the identification" (based on which processor 220 determines the many-valued quality score) refers to the information generated by processor 220 during the identification of the parts of the physiological data which result from the physiological process. The "results of the identification" indicate which parts of the physiological data result from the physiological process (and possibly also which parts of the physiological data do not result from the physiological process).

Significantly, the many-valued quality scores determined by processor 220 are indicative of a suitability of the physiological data for the analysis of the specific physiological process (e.g. for diagnosing a specific medical condition of the patient). General quality scores may be used to describe the general quality of the signal (e.g. volume level, overall luminance of the picture, signal to noise ratio). However, such general quality scores do not provide sufficient information for assessing the suitability of the physiological data for the analysis of any specific physiological process (e.g. for diagnosing any specific medical condition of the patient).

For example - an image collected by a camera may be very detailed, well lighted and focused - but if it does not capture a good view on the tonsils - it cannot be used to assess the condition of the tonsils (e.g. in order to identify throat diseases). The many-valued quality score determined by processor 220 in such a case may further depend, for example, on the relative portion of the tonsils shown in the image (based on image processing of the image), on the focus on the tonsils (based on respective image processing), on the color correctness of the tonsils area, and so on.

The many-valued quality score for image or video may take into account, for example, a degree of an object associated with the physiological process (e.g. tonsils, mole, or eardrum) being located within the field-of-view (FOV) (e.g. what portion of the tonsils/mole/eardrum/etc. is visible in the image/video) and a degree of the object being in focus. Additional factors may include, for example, that the image/video of the object is sufficiently stable, well lighted, in the correct distance, visible for sufficient time duration, etc.

In another example, a sound sample collected by a digital stethoscope may collect a high volume low noise signal - but this signal may be a good quality signal of another physiological process or even of ambient sound (e.g. other people talking in the room, or even the patient herself talking, friction of the system 200 over the patient's body or clothes, etc.). Such a signal may not be useful, for example, for analysis of the breathing and of the condition of the lungs. The many-valued quality score determined by processor 220 in such a case may further depend, for example, on identification of rhythmical (or arhythmical) breathing patterns, of removing identifiable heartbeats sounds from the signal of the physiological data, and so on. The many-valued quality score for audio signal may include, for example, identifying that the relevant parts of the signal (i.e. which capture the physiological process) are of sufficient time duration, have enough amplitude ratio with respect to other parts of the signal (e.g. noise or other sources), and so on.

Processor 220 may be configured in different ways to determine the many-valued quality score based on the physiological data and on results of the identification. Especially, processor 220 may be configured to determine the many-valued quality score based on parts of the physiological data which it identified as resulting from the physiological process (one or more of these parts), and optionally also on other parts of the physiological data (e.g. on parts of the physiological data which processor 220 identified as resulting from other processes, from ambient signals, or generally from other sources).

Some examples of ways in which the results of the identification and the physiological data itself may be used by processor 220 for the determining of the many-valued quality scores are:
a. The many-valued quality score may be determined based on the magnitude (e.g. amplitude) of parts of the physiological data determined to result from the physiological process;
b. The many-valued quality score may be determined based on a ratio between parts of the physiological data determined to result from the physiological process and other parts of the physiological data;
c. The many-valued quality score may be determined based on a ratio between magnitudes of parts of the physiological data determined to result from the physiological process and magnitudes of other parts of the physiological data;
d. The many-valued quality score may be determined based on cumulative amount of parts of the physiological data determined to result from the physiological process (e.g. enough times in which data resulting from the physiological process is of sufficient quality);
e. In cases where the physiological data is an image or a video stream, the many-valued quality score may be determined based on visibility of a certain organ within the physiological data or a relative portion of such organ within the physiological data (e.g. the more such organ is visible within the physiological data - the higher the grade), or existence of one or more specific markers (natural and/or artificial) within the physiological data, etc.
f. Etc.

It is noted that processor 220 may be operable to determine many-valued quality scores of more than one type - either for analysis of different physiological processes, or for analysis by different entities. For example, one type of quality score may be used if the analysis is intended to be executed by a human physician, while another type of quality score may be used if the signal (the physiological data or part thereof) is intended to be analyzed by a dedicated computerized system. It is noted that the different types of quality scores are used (and hence also selected during or prior to) during the time of measuring the physiological data - e.g. in order to enable the collection of data which is useful for analysis by the specific analyzing or diagnosing entity and/or for analysis or diagnosis of a preselected physiological process. Several degrees of quality levels or scales may be used by processor 220 and by other components of system 200.

Processor 220 can be further configured to provide (at different times during the physiological measurement) a many-valued quality-feedback information which is based on the quality score. Since the many-valued quality-feedback information is based on the many-valued quality score, the many-valued quality-feedback information is also indicative of the suitability of the physiological data for the analysis of the physiological process.

The many-valued quality-feedback information may be identical to a many-valued quality score determined by processor 220, or otherwise based on such value (many-valued quality score). For example, processor 220 may determine at one point in time a decimal value between 0.01 and 100.00 as a many-valued quality score, but the many-valued quality-feedback information may be provided by a seven LED (light emitting diodes) scale, where the number of lit LEDs indicate the quality level of the obtained physiological data for which the many-valued quality score was determined (i.e. the suitability of the collected data for analysis of the physiological process).

Processor 220 provides the many-valued quality-feedback information using tangible user interface 230 (also referred to as UI 230) of system 200. Different kinds of user interfaces 230 may be used for the providing of the many-valued quality-feedback information. In some cases, the user interface 230 can be part of a handheld medical device operated by the user (e.g. a display, a speaker, one or more vibrating elements, a group of LEDs, etc.). Additionally, or alternatively, the user interface 230 can be external to a handheld medical device operated by the user, such as an external display, an output means of a smartphone (a smartphone display, speaker, vibrating elements, etc.) or another computer (where the information can be provided on a user interface of that computer) and so on (in such cases, the many-valued quality-feedback information can be provided to such external user interface via a wired/wireless connection). It is noted that UI 230 may optionally be used to provide additional information to a user of system 200, whether originating from processor 220 or not. For example, UI 230 may optionally additionally provide instructions for how to change measurement for improving quality of the measurement, for indicating an end of measurement (or measurement part, e.g. moving to another location on the body to continue the measurement) and so on. Such additional information may optionally be provided by UI 230 during the examination, on not only after it concludes. However, it is not necessary that any information (whether the many-valued quality-feedback information or any other information provided by UI 230) would be provided at all times (or at any specific time) throughout the examination.

Optionally, processor 220 may be configured to generate the analysis source data based on at least one of the many-valued quality scores and on the physiological data obtained at at least one of the plurality of different times. For example, processor 220 may choose to include in the analysis source data only information from some of the measured moments (e.g. when the quality scores indicated high quality) but not from other times. For example, processor 220 may process the collected data to remove (or reduce) data which results from other physiological processes, from ambient signals, or from any other sources except the physiological process which is intended for analysis. Other ways of generating the analysis source data based on one or more of the quality scores and on the physiological data, in order to generate analysis source data which is better suitable for analysis of the specific physiological process, may also be used. It is noted that other processing of the same physiological data would be implemented by processor 220 in order to generate analysis source data for analysis of another physiological process. It is noted that the analysis source data can be different than the physiological data (e.g. it optionally contains less/other/additional data than the physiological data, etc.).

Although reference was made to analysis of physiological processes, it is to be noted that system 200 can also be configured to operate on an instantaneously acquired physiological data, such as an image of the patient or a body part thereof, a blood sample of the patient, or any other instantaneously acquire physical data. In such cases, the processor 220 can be configured to analyze the instantaneously acquire physical data to determine presence of diagnosis-enabling data, as further detailed herein. Diagnosis-enabling data is data that enables a diagnosing entity (e.g. a medical practitioner, a computerized diagnosis system, etc.), to later diagnose a medical condition of the patient from which the instantaneously acquire physical data originates. The processor 220 can be configured to provide the user of the system 200 with feedback, e.g. in the form of an indication of presence/absence of diagnosis-enabling data within the instantaneously acquire physiological data.

Fig. 2 is a functional block diagram illustrating an example of system 200, in accordance with the presently disclosed subject matter. It is noted that system 200 may include one or more physiological measurement sensors 210, as well as additional components such as one or more of the following modules: communication module 240 (enabling wired and/or wireless communication with external devices), power source 250, casing 260, and so on.

Few examples of physiological sensors 210 are illustrated in Fig. 2: camera 211 (denoted CAM 211), two microphones 212 (denoted MIC 212), Thermometer 213 (denoted TMP 213). Optionally camera 211 may be operable to capture visible light, and to generate images based on light it captures. Camera 211 may also be sensitive to other parts of the electromagnetic spectrum near the visible spectrum (e.g. to infrared radiation, such as near IR radiation), but this is not necessarily so. It is nevertheless noted that other types of sensors 210 and other combination of sensors 210 may be implemented, e.g. as discussed above in greater detail. For example, the physiological sensors 210 can include blood pressure sensor/s e.g. for measuring a blood pressure of the patient, one or more accelerometers for measuring movements of the system 200, pressure sensors for determining an amount of pressure exerted by the system 200 on the patient's body, etc.

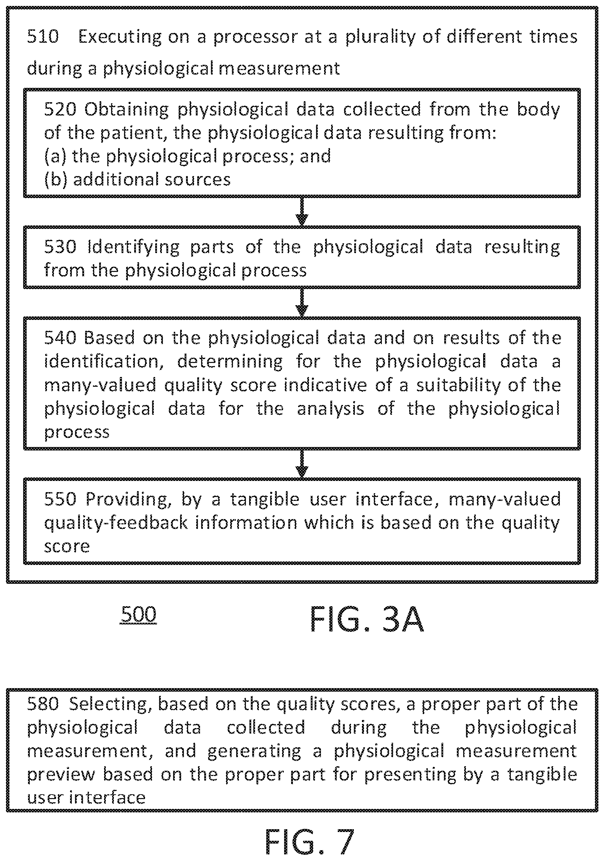

Fig. 3A is a flow chart illustrating an example of method 500, in accordance with the presently disclosed subject matter. Method 500 is a method for providing feedback indicative of a suitability of data, collected during a physiological measurement, for analysis of a physiological process of a body of a patient. Referring to the examples set forth with respect to the previous drawings, method 500 may be executed by system 200. Any variation, combination or optional implementation which is discussed with respect to system 200 may be implemented, mutatis mutandis, also with respect to method 500. Any variation, combination or optional implementation which is discussed with respect to method 500 may be implemented, mutatis mutandis, also with respect to system 200. As described below in detail, method 500 may be used for preparing analysis source data for analysis of a physiological process of a body of a patient.

It is noted that the patient and/or any other person or conductor of the measurement does not necessarily have to be informed about the target of the measurement, or which specific parameter it is intended to measure for further analysis/diagnosis.

Stage 510 of method 500 is executed during a physiological measurement, and includes executing on a processor at a plurality of different times during the physiological measurement the stages 520, 530, 540 and 550 of method 500 at a plurality of different times during the physiological measurement. Referring to the examples set forth with respect to the previous drawings, stage 510 (or one or more substages of which, including any combination of stages 520, 530, 540 and optionally also 550) may be executed by processor 220.

Stage 520 includes obtaining physiological data collected from the body of the patient, the physiological data resulting from: (a) the physiological process and from (b) additional sources. As discussed with respect to system 200, the physiological data collected at stage 520 may be collected by one or more sensors.

It is noted that optionally, stage 520 may include obtaining physiological data which is collected by physiological sensors which are not directly connected to the unit which executes stage 520. For example, stage 520 (and optionally the entire stage 510) may optionally be executed by a processor of a smartphone or another multi-purpose computer or a dedicated computer (e.g. a laptop computer, a server, a medical application computer, and so on), while the collection of the physiological data is executed by a portable (optionally handheld) unit which is operated by the patient or by someone in the vicinity of the patient (e.g. in a distance that enables such person to manually operate the portable unit for acquiring the physiological data).

Nevertheless, the collection of the physiological data may optionally be executed by one or more physiological sensors which are connected to the processor which executes stage 520 via a mechanical connection, a wired connection, a wireless connection, and so on. Optionally, method 500 may include stage 505 of collecting the physiological data from the body of the patient. Stage 505, if implemented, is also executed at different times during the physiological measurement. Referring to the examples set forth with respect to the previous drawings, stage 505 may be executed by one or more sensors 210 and/or by one or more external sensors.

Stage 530 includes identifying parts of the physiological data resulting from the physiological process. Additional information regarding how the identification may be achieved are discussed with respect to processor 220, above.

Stage 530 may be executed using different types of algorithms and/or electric circuits (including digital signal processing and/or analog signal processing). It is noted that the identification of the parts of the physiological data which result from the physiological process may include identifying the physiological process (e.g. heartbeats) but may also include (in addition or instead) identifying other parts of the physiological data (e.g. other physiological processes such as breathing, ambient signals, etc.). The identification of other parts of the physiological data (which do not result from the physiological process) may be used to eliminate such part and/or to assist in identifying the parts of the physiological data which result from the physiological process.

It is noted that stage 530 may be based on identification of effects of a plurality of different physiological processes on the physiological data. For example, stage 530 may include identifying in the sound sample of the physiological data both the heartbeats and the sounds of the breathing.

As indicated herein, in some cases, the physiological data obtained at stage 520 can result from the physiological process and from additional sources. In some cases, the additional sources can include ambient signals. In such cases, the physiological data can be analyzed for determining if the ambient signals exceed a threshold, and if so - an alert can be provided to the user. In some cases, the processor 220 can further provide the user with an indication of a cause of the ambient sound (e.g. an indication that the ambient sound is people talking, an air conditioner making noise, friction of the system 200 over the patient's body or clothes, etc.). The cause of the ambient sound can be determined, for example, using filters, Mel-Frequency Cepstrum (MFC), Short-Time Fourier Transform (STFT), or other known statistical measures such as comparing the variance in both frequency domain and time domain, generating Gaussian mixture model for voice and non-voice, or other methods and/or techniques known in the art (e.g. SOHN, A statistical model-based voice activity detection IEEE Signal Processing Letters ( Volume: 6, Issue: 1, Jan. 1999) or usage of "VOICEBOX" (Speech Processing Toolbox for MATLAB) and/or other standard machine learning models (such as Support Vector Machine (SVM) or other techniques). In some cases, the processor 220 can be configured to identify ambient signal exceeding a threshold and alert the user before obtaining the physiological data at stage 520.

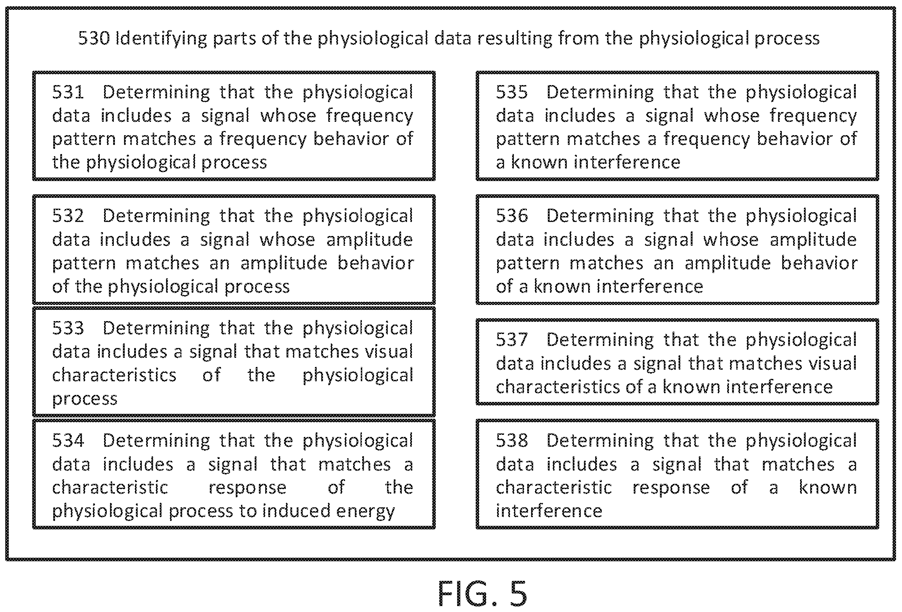

Fig. 5 illustrates optional ways of implementing stage 530, in accordance with examples of the presently disclose subject matter. Stage 530 may include any combination of one or more of stages 531 through 538.

Stage 531 includes determining that the physiological data includes a signal whose frequency pattern matches a frequency behavior of the physiological process. Stage 531 may be executed based on predetermined parameters characterizing the frequency behavior of the physiological process. The frequency may be a temporal frequency (e.g. cycles per seconds), spatial frequency (e.g. cycles per millimeter), or a combination of both. For example, repeating pattern created by the physiological process in the collected physiological data can be searched, for example detecting heart beats (as S1, 52) which has a typical repetitive nature, to detect that the collected physiological data include diagnosis-enabling data. Detection of this phenomena can be made, for example, by training a classifier (based on positive and negative examples) to detect S1 and 52. Given an input signal, a multiscale sliding window can be used for obtaining samples from the signal, that can be classified using the classifier. Finally, the samples that yielded the K highest scores classified by the classifier (if they're above a specific threshold) are cross correlated with the whole signal. Peaks in the cross-correlation results are indicative of the quality of the heart signal.

Stage 532 includes determining that the physiological data includes a signal whose amplitude pattern matches an amplitude behavior of the physiological process. Stage 532 may be executed based on predetermined parameters characterizing the amplitude behavior of the physiological process. The amplitude pattern may be time dependent or not, frequency dependent or not, and so on.

Stage 533 includes determining that the physiological data includes a signal that matches visual characteristics of the physiological process. Stage 533 may be executed based on predetermined parameters characterizing the visual characteristics of the physiological process. The visual characteristics may relate, for example, to visual resemblance, characteristic lighting patterns, characteristic color patterns, characteristic contrast between parts, and so on. It is noted that the visual characteristic may pertain to the visible spectrum, or to other parts of the electromagnetic spectrum. It is noted that the visual characteristic may pertain to the behavior of physiological body elements of the patient under active lighting provided by an artificial system, or under regular light.

Stage 534 includes determining that the physiological data includes a signal that matches a characteristic response of the physiological process to induced energy (e.g. ultrasound waves, mechanical pressure, electric current, and so on). Stage 534 may be executed based on predetermined parameters characterizing such a characteristic response of the physiological process.

Stage 535 includes determining that the physiological data includes a signal whose frequency pattern matches a frequency behavior of a known interference. The term "known interference" pertains to another physiological process, to a known ambient sound (e.g. characteristic noise of the sensor, of ambient environment, of the voice of the patient, friction of the system 200 over the patient's body or clothes, etc.), or another known source of signal (e.g. compensating for lighting other than that issued by the measuring system). Stage 535 may be executed based on predetermined parameters characterizing the frequency behavior of the known interference. The frequency may be a temporal frequency (e.g. cycles per seconds), spatial frequency (e.g. cycles per millimeter), or a combination of both.

Stage 536 includes determining that the physiological data includes a signal whose amplitude pattern matches an amplitude behavior of a known interference. Stage 536 may be executed based on predetermined parameters characterizing the amplitude behavior of the known interference. The amplitude pattern may be time dependent or not, frequency dependent or not, and so on.

Stage 537 includes determining that the physiological data includes a signal that matches visual characteristics of a known interference. Stage 537 may be executed based on predetermined parameters characterizing the visual characteristics of the known interference. The visual characteristics may relate, for example, to visual resemblance, characteristic lighting patterns, characteristic color patterns, characteristic contrast between parts, and so on. It is noted that the visual characteristic may pertain to the visible spectrum, or to other parts of the electromagnetic spectrum. It is noted that the visual characteristic may pertain to the visual behavior under active lighting provided by an artificial system, or under regular light.

Stage 538 includes determining that the physiological data includes a signal that matches a characteristic response of a known interference to induced energy (e.g. ultrasound waves, mechanical pressure, electric current, and so on). Stage 538 may be executed based on predetermined parameters characterizing such a characteristic response of the known interference.

It is to be noted that in some cases, various machine learning models (such as Support Vector Machine (SVM) or other techniques) can be used for determining presence of diagnosis-enabling data (e.g. existence of specific forms in an image (e.g. tonsil, tympanic membrane, body parts) or specific segments in audio (e.g. S1, S2 in heart) within the physiological data.

It is to be noted that these stages are mere examples and many other ways of implementing stage 530 are contemplated as well.

Reverting to Fig. 3A, stage 540 includes determining for the physiological data, based on the physiological data and on results of the identification, a many-valued quality score indicative of a suitability of the physiological data for the analysis of the physiological process. Additional information regarding how the many-valued quality score may be determined are discussed above with respect to processor 220.

The "Results of the identification" (based on which the many-valued quality score is determined in stage 540)) refers to the information generated at stage 530, which indicate which parts of the physiological data result from the physiological process (and possibly also which parts of the physiological data do not result from the physiological process).

Optionally, the quality score (i.e. the many-value quality score) determined for the physiological data at stage 540 may be different than any corresponding value included in the analysis source data (i.e. it may be different than the amplitude, the volume, etc., at the part of the physiological data for which the quality score is determined). Optionally, the quality score (i.e. the many-value quality score) determined for the physiological data at stage 540 may be different than any value included in the analysis source data.

It should be noted that different types of quality scores may be determined - e.g. scalar, vector, etc. Optionally, more than a single many-valued quality score may be determined for physiological data collected at any one or more of the times. The plurality of many-valued quality scores - if so determined - may be stored as a vector, as a plurality of variables, or in any other suitable way. Optionally, the determining of stage 540 includes determining two or more many-valued quality scores (where each of the scores is many-valued, i.e. more than just pass/fail or other binary representation). The determining of a plurality of quality scores - if implemented - may be executed for all of the physiological data collected throughout the entire physiological measurement, but this is not necessarily so.

The determining of the many-valued quality score in stage 540 may be based on the physiological data and on results of the identification in different ways. Especially, the many-valued quality score determined in stage 540 is based on parts of the physiological data which were identified in stage 530 as resulting from the physiological process (one or more of these parts), and may optionally be further based on other parts of the physiological data (e.g. on parts identified in stage 530 as resulting from other processes, from ambient signals, or generally from other sources).

Some examples of ways in which the results of the identification and the physiological data itself may be used in stage 540 are:
a. The many-valued quality score may be determined based on the magnitude (e.g. amplitude) of parts of the physiological data determined to result from the physiological process;
b. The many-valued quality score may be determined based on a ratio between parts of the physiological data determined to result from the physiological process and other parts of the physiological data;
c. The many-valued quality score may be determined based on a ratio between magnitudes of parts of the physiological data determined to result from the physiological process and magnitudes of other parts of the physiological data;
d. The many-valued quality score may be determined based on cumulative amount of parts of the physiological data determined to result from the physiological process (e.g. enough times in which data resulting from the physiological process is of sufficient quality);
e. In cases where the physiological data is an image or a video stream, the many-valued quality score may be determined based on visibility of a certain organ within the physiological data or a relative portion of such organ within the physiological data (e.g. the more such organ is visible within the physiological data - the higher the grade), or existence of one or more specific markers (natural and/or artificial) within the physiological data, etc.
f. Etc.

The determining of the many-valued quality score may optionally be based on a selection of a scoring scheme out of a plurality of predefined scoring schemes, wherein each scoring scheme is associated with an analysis process for the physiological process. For example, a certain sample (physiological data, e.g. video of the ear canal) may be sufficient for preliminary analysis (e.g. determining color of ear canal or rupture of the eardrum) but not for detailed analysis (e.g. analyzing the state of an ear fungus). Different quality scores can be given to the same sample, based on a scoring scheme. Different scoring schemes may be used, for example, if the collected physiological data should be used (e.g. for analysis/diagnosis) by a person (e.g. a physician) or by a computerized system. The selection of the scoring scheme can be done during the physiological examination, or before it.

It is noted that the many-valued quality scores may be indicative of a degree in which the patient follows instructions for physical activities. For example, during auscultation of the lungs, the patient may be instructed to breath in different ways (e.g. in, out, hold, deep, etc.), or to hold the sensor in a stable position over the patient skin during the measurement in order to attempt increasing the many-valued quality score) when it is subsequently determined (e.g. at a second time later than a first time for which a many-valued quality score was determined indicative of a potential for improving the reading by implementing the instruction provided to the user, the second time being after the patient starts acting according to the instruction). A many-valued quality score may be determined for physiological data as an assessment of the degree to which the patient followed the instructions.

The many-valued quality score may be determined for physiological data collected by one or more sensors, based in part on physiological (or other) data collected by one or more other sensors. For example, blood pressure measurement may be assigned a many-valued quality score which is also based on an assessment of the patient following her breath pattern instructions - which may be determined by auscultation.

It is noted that optionally, the determining of the many-valued quality score at stage 540 may be further based on data collected by non-physiological sensor of a physiological measurement system which collected the physiological data. The non-physiological sensor may optionally collect data relating to the environment (e.g. microphone sampling ambient sound, light sensor measuring light level of surrounding environment, ambient temperature thermometer, ambient humidity level sensor, and so on). The non-physiological sensor may optionally collect data relating to a state of the measuring system which includes the physiological sensor collecting the physiological data. For example, such non-physiological sensor may be an inertia measurement unit (IMU) which measures movement of the sensor in one or more dimensions (whether translation and/or rotation of the system). For example, such non-physiological sensor may measure the temperature of the measurement system (or specific part or parts thereof), the state of its subsystems, etc.

It is noted that the determining of the many-valued quality score at stage 540 may also be implemented based only on the physiological data and on results of the identification, without any additional data. It is noted that the determining of the many-valued quality score at stage 540 may also be implemented based on the physiological data and on results of the identification, without any additional measurement data (but possibly using some other forms of data such as clock data, etc.).

Optionally, stage 540 may include determining the many-valued quality score based on criteria determined by an expert (e.g. physician, technician) at a remote location. Optionally, the criteria may be determined by the expert (e.g. physician, technician) during the physiological measurement, possibly based on data previously collected at an earlier time of the physiological measurement. For example, the expert may indicate points of interest (POI), such as specific locations in the body, specific range of acoustic measurement data, and so on.

Stage 550 includes providing, by a tangible user interface, many-valued quality-feedback information which is based on the quality score (which is, as mentioned above, a many-valued quality score). Referring to the examples set forth with respect to the previous drawings, stage 550 may be executed by processor 220, by UI 230 or by a combination of both. It is noted that the tangible user interface of stage 550 may be part of the same system to which the processor which executes stage 550 belongs, but this is not necessarily so. For example, a portable hand-held physiological monitoring unit may collect the physiological data and process it, and then send the feedback information to be provided by another system (e.g. the UI of a smartphone of the patient, of another computer in the room, a wireless speaker, etc.).

The quality feedback information provided in stage 550 may be identical to one or more of the quality scores determined in stage 540, or another information based on such one or more quality score. Optionally, the quality feedback information provided at stage 550 may be different than any corresponding value included in the analysis source data (i.e. it may be different than the amplitude, the volume, etc., at the part of the physiological data for which the quality score is determined). Optionally, the quality feedback information provided at stage 550 may be different than any value included in the analysis source data.

It is noted that optionally, the physiological data is collected by a physiological measurement device (e.g. system 200), and the suitability of the physiological data changes as a result of changes in operating of the physiological measurement device by a user which perceives the quality feedback information presented by the tangible user interface. The quality of the physiological measurement can be affected by changes to the process which are manifested by the user (e.g. the patient or a person in its vicinity), which receives feedback and acts upon it. The changes in operating of the physiological measurement device by the user can include one or more of: moving or readjusting the position/orientation of the measurement unit (e.g. the sensor/s, or a unit comprising the sensors, used for acquiring the physiological data), applying more pressure by the sensor/s on the patient's body, changing one or more measurement parameters, readjusting the patient's body position, breathing differently, replacing modules (e.g. otoscope speculum) of the measurement unit, or any other way discussed herein. It is noted that in addition to the many-valued quality feedback information, additional information may be provided to the user, in order to improve the process of measurement based on data which is already collected and its suitability for analysis of the specific physiological process.

It is noted that method 500 may include providing of additional information using the UI - to the patient and/or to a person/system which controls the examination (if it is not the same person).

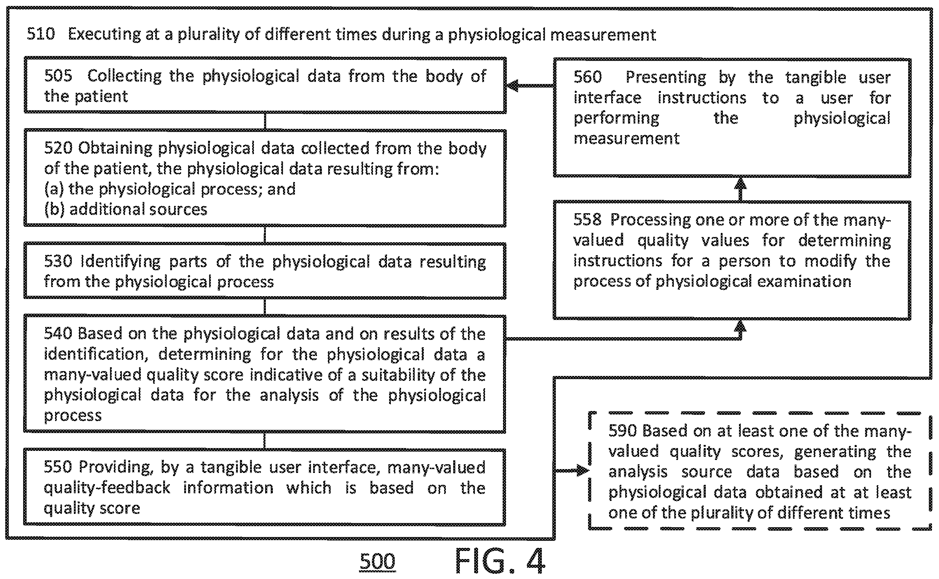

Referring to the example of Fig. 4 (which is a flow chart illustrating an example of method 500, in accordance with the presently disclosed subject matter), it is noted that stage 510 may also include stage 560 of presenting by the tangible user interface instructions to a user for performing the physiological measurement. For example, stage 560 may include providing by the tangible UI instructions for modifying the procedure of physiological examination, where the instructions are determined as part of method 500 based on one or more of the many-valued quality scores.

The feedback and/or instructions provided during method 500 may optionally include information regarding possible causes for interference or degradations of the collected physiological data. The feedback and/or instructions provided during method 500 may optionally include information regarding actions which may be taken by a user of the system in order to overcome such interferences or possible causes for degradations of the collected physiological data.

Stage 560 may be preceded by stage 558 of processing one or more of the many-valued quality scores for determining instructions for a person to modify the process of physiological examination. Referring to the examples set forth with respect to the previous drawings, stage 558 may be executed by processor 220.

For example (referring to stages 558 and/or 560), the instructions may pertain to the positioning of the physiological sensor which collects the physiological data, to the operational parameter of the measurement device (e.g. system 200) and/or of the physiological sensor, to actions the patient should do (e.g. hold her breath, cough, stand up and turn around, etc.), to the environment (e.g. reduce ambient sound/light), and so on. The UI may also be used as part of method 500 for indicating an end of measurement - or of a stage or part of the measurement (e.g. end of medical examination or end of a specific part of the medical examination in case the medical examination has several parts, etc.).

It is noted that stages 558 and/or 560 may be carried out once during the entire physiological measurement, or more than once. For example, instructions may be generated only when the many-valued quality score falls below a predetermined threshold, or falls below a predetermined threshold for a predetermined amount of time.

Referring to stage 510 as a whole, it is noted that while each of the substages of stage 510 are executed at different times during the physiological measurement, the number of time each of the substages is not necessarily identical. For example, stage 520 may be executed practically continuously (e.g. collecting 400 samples per second), stage 530 may be executed at a lower rate (e.g. every second, every 25 samples, etc.), stage 540 in yet another rate (e.g. every half a second), and so on. Each substage of stage 510 (e.g. stage 530, 560, etc.) may be based on one iteration of a previous substage, or on more than one iteration. It is noted that different substages of stage 510 may be executed concurrently, but this is not necessarily so. For example, at a specific time, a quality score may be determined for physiological data collected a second ago, while new physiological data is being collected concurrently.

As discussed with respect to system 200 and to method 500, some actions are executed at a plurality of times throughout the physiological measurement. Optionally, that plurality of times may include at least a first time (e.g. a first moment or a first span of time) and a second time (e.g. a second moment other than the first moment or a second span of time other than the first span of time). The second time is later than the first time, however, in case of a first and a second span of time, the first and second span of time can optionally partially overlap. Optionally, the obtaining of the physiological data at the second time (e.g. at stage 520) is affected by changes of the physiological measurement by the user as a result from providing by the tangible user interface of the many-valued quality feedback information resulting from many-valued determined for physiological data obtained at the first time. The changes of the physiological measurement can include one or more of: moving or readjusting the position/orientation of the measurement unit (e.g. the sensor/s, or a unit comprising the sensors, used for acquiring the physiological data), applying more pressure by the sensor/s on the patient's body, changing one or more measurement parameters, readjusting the patient's body position, breathing differently, avoiding friction of the system 200 over the patient's body or clothes, replacing modules (e.g. otoscope speculum) of the measurement unit, or any other way discussed herein.

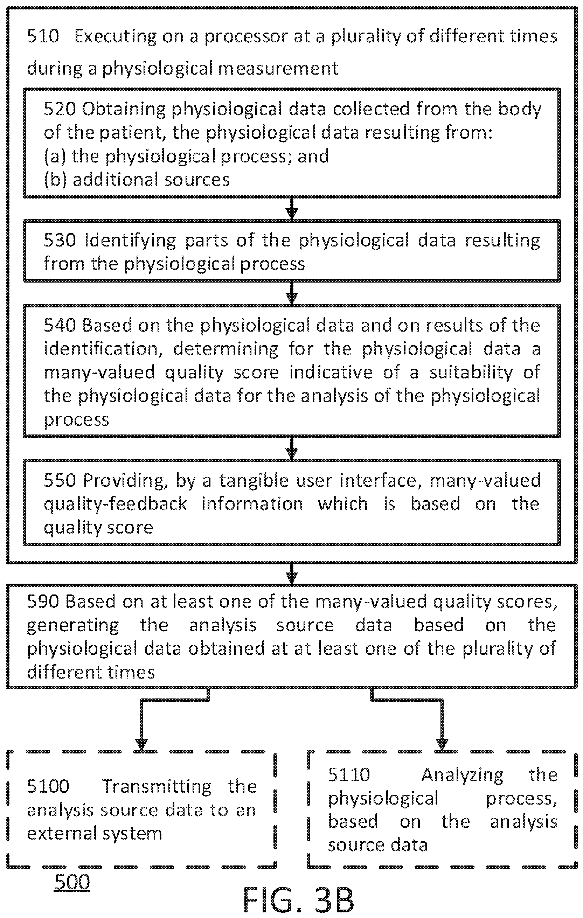

Fig. 3B is a flow chart illustrating an example of method 500, adapted for preparing analysis source data for analysis of a physiological process of a body of a patient, in accordance with the presently disclosed subject matter. Method 500 may also include stage 590 which includes generating the analysis source data (which includes at least diagnosis-enabling data) based on at least one of the many-valued quality scores and based on the physiological data obtained at at least one of the plurality of different times. Referring to the examples set forth with respect to the previous drawings, stage 590 may be executed by processor 220. It is noted that stage 590 may also be executed by a processor of a separate system. Stage 590 may be executed after stage 510 is completely executed, or partly concurrently to stage 510. That is, part of the analysis source data may be generated before the collection and/or processing of the physiological data at stage 510 are concluded - but this is not necessarily so. Additional information regarding how the generation of the analysis source data may be achieved are discussed with respect to processor 220, above.

Referring to stage 590, it is noted that optionally the generating of the analysis source data may include compressing different parts of the physiological data based on different many-valued quality scores determined for the different parts. in some cases, parts of the physiological data which were assigned a low-quality score may be compressed using higher compression level (and/or lower preservation rate of compression) with respect to parts which received higher quality scores. It is noted that some parts may be omitted from the analysis source data altogether (e.g. if their many-valued quality score indicates irrelevancy or unsuitability for analysis - e.g. because the signal is of inferior quality, because it does not include information of a relevant body part).

Stage 590 may include generating analysis source data which includes metadata that indicates times during the physiological measurement during which measurements of higher quality where obtained. Examples of higher quality include less noise, better ratio of the signal of the physiological process with respect to other signals in the measurements, etc. Such metadata may include ranking for different points in time, it may include indications of time durations (e.g. between 5.51 seconds to 9.54 seconds into the measurement) in which highest quality measurements were obtained, and so on.

It is noted that the generating of the metadata for the analysis source data may use parameters which are time accumulated. For example, times during the physiological measurement may be marked as being of high quality if there is continuous measurement which qualify a certain condition (e.g. eardrum is visible) for at least a predetermined duration (e.g. for at least 3 continuous seconds). For example, times during the physiological measurement may be marked as being of high quality if there is accumulated measurement which qualify a certain condition (e.g. heart sounds can be heard) for at least a predetermined accumulative duration (e.g. for at least 10 seconds, not necessarily consecutive).

Method 500 may also include optional stage 5100 of transmitting the analysis source data (including at least diagnosis-enabling data) to an external system. The transmitting may include transmitting the analysis source data to a system which will analyze the data, or to any other system (e.g. a storage server, for later use). The transmitting of stage 5100 may include transmitting the information wirelessly, over cable connection, or in any other way.

Optionally, stage 5100 may include transmitting the analysis source data which includes the compressed physiological data to the external system.

Optionally, method 500 may include stage 5110 of analyzing the physiological process (e.g. diagnosing a medical condition of the patient based on the physiological data relating to the physiological process), based on the analysis source data. Referring to the examples set forth with respect to the previous drawings, stage 5110 may be executed by processor 220.

As mentioned above, sometimes the apparatus that collects the physiological data can collect physiological data intended to be used for analysis of different physiological processes, or even for different types of analysis of a single physiological process. Likewise, sometimes the apparatus which analyzes the collected data (whether it is the same apparatus or not) is capable of analyzing - using the collected data - different physiological process, or even apply different form of analysis to a single physiological process.

In such cases, the determining of the many-valued quality score for the collected physiological data can depend on selection of which analysis is intended to be executed. The determining of the many-valued quality score may optionally further be based on parameters of an analysis procedure selected out of a predefined finite plurality of analysis procedures for analyzing the physiological data.

The selection of which analysis process is the target of data collection and/or the selection of parameters may be done by a person operating the machine, automatically (e.g. based on data of sensor, e.g. camera data may be used to determine proximity to a specific organ, on which the selection may be based), or received from an external system (e.g. a server, a physician's station, and so on).

So, while the apparatus may collect physiological data which may be useful also for other measurements and/or analysis processes, the scoring (the determination of the many-valued quality score) may be determined based on the goal of the specific measurement.

For example, the apparatus used in method 500 (e.g. system 200) may be used at one time for auscultation of the heart (recording sounds originating from the heart of the patient, where the operation of the heart is the monitored physiological process), and on another time for auscultation of the lungs (recording sounds originating from the lungs of the patient, where the operation of the lungs is the monitored physiological process). The selection of what is the physiological process and/or what sort of analysis the collected physiological data will be used for may be made by a user, automatically, or by a remote system. It is noted that this selection may change in different times.

It is noted that stage 590 is different than general-purpose noise reduction at least in that it is specific for the preparation of high quality data for analysis of a specific physiological process. A very clean signal may not contain enough information which is relevant for analysis of the specific physiological process, as exemplified above. In comparison, method 500 enables generation of the analysis source data while monitoring the suitability of the collected data for analysis of the specific process, and making the required adjustments in order to make sure that the analysis source data based on the collected data is suitable for this specific purpose - i.e. that it includes diagnosis-enabling data.

Referring to method 500 as a whole, it is noted that optionally, the obtaining (stage 520), identifying (stages 530) and determining (stage 540) are executed by a portable handheld physiological monitoring device. In such a case, the obtaining (stage 520) may include measuring the physiological measurement by at least one physiological sensor of the portable handheld physiological monitoring device (stage 505, illustrated as a separated stage as a matter of convenience). It is noted that while all of those stages may be executed by the physiological measurement device (whether portable or not) which collects the data, this is not necessarily so - and some or all of these stages may also be executed on another system - e.g. a personal computer, a smartphone, a server, a remote computer (e.g. a physician's station), etc.

It is noted that optionally, the obtaining (520), processing (530), determining (540) and providing (550) are repeated for a plurality of successful physiological measurements, while the determining includes different many-value quality scores (i.e. different quality assessments) to different successful physiological measurements. A successful physiological measurement, for the context of the present discussion, means a measurement which qualifies a predetermined condition, which is sufficient for medical analysis/diagnosis (i.e. the measurement includes diagnosis-enabling data) and which is saved and displayed to a user (e.g. a physician). By giving different scores to different successful measurements - the operator of the measurement has a chance to improve the quality of measurement, to make it more efficient (e.g. shorter), and so on. Giving feedback on quality and not only on success/failure allows to educate the patient/operator.

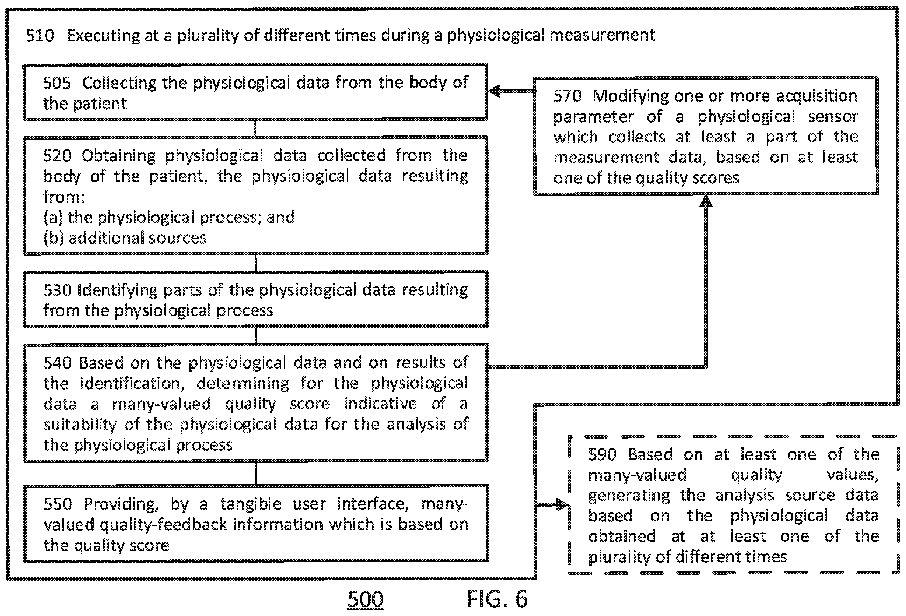

Fig. 6 is a flow chart illustrating an example of method 500 in which the determination of the many-valued quality scores are used for automatically modifying acquisition parameters used in the acquisition of the physiological data, in accordance with the presently disclosed subject matter.

Method 500 may therefore further include stage 570 of modifying one or more acquisition parameter of a physiological sensor which collects at least a part of the measurement data, based on at least one of the quality scores. Referring to the examples set forth with respect to the previous drawings, stage 570 may be executed - or at least controlled - by processor 220. Referring to the examples set forth with respect to the previous drawings, the acquisition parameters may be parameters of sensor 210 (and also, in some cases, of processor 220 or other components of system 200).

Several non-limiting examples of acquisition parameters which can be modified in stage 570 include:
a. Temporal parameters (e.g. acquisition frequency, sampling rate, duration, timing, etc.);
b. Electrical parameters (resistance, applied current, voltage, etc.);
c. Physical parameters (e.g. sampling temperature, etc.);
d. Camera parameters (e.g. lighting threshold, white balance, contrast, focus, focal point, etc.);
e. Microphone parameters (e.g. frequency based filtering such as high pass, low pass, band pass, band stop, etc., sampling volume, sampling sensitivity, etc.);
f. Positioning parameters (moving with respect to the body, if possible);
g. Sensor selection (e.g. if two or more similar sensors are used);
h. Preprocessing parameters (e.g. parameters of noise-reduction sensitivity, etc.);
i. And so on.

It is noted that stage 570 may be carried out once during the entire physiological measurement, or more than once. For example, modification of acquisition parameters may be required only when the many-valued quality score falls below a predetermined threshold, or falls below a predetermined threshold for a predetermined amount of time, consecutively or accumulatively. Optionally, stages 560 and 570 may be implemented synergistically. For example, modification of acquisition parameters may be most effectively utilized if the user would move the sensor; other changes by the user (instructed or not) may require modification of acquisition parameters.

In addition to the many-valued quality score (or scores), the acquisition parameter may be modified in stage 570 based on additional factors - such as patient parameters, sensor parameters, environment parameters, etc. The other parameters may be used for determination of the new acquisition parameter (e.g. the new sampling frequency) and/or for determination of a quality score threshold (or other criterion) for deciding when modification of the acquisition parameter is required.

Optionally, the modifying of the acquisition parameter is executed further in response to a medical condition of the patient. The medical condition (or more generally - physiological condition of the patient) may be a prolonged condition (such as body weight, diabetes, normal blood pressure, etc.), or a more transient medical condition (e.g. illness such as sour throat, fever etc.). For example, optimal sound quality for auscultation may depend on fat-concentrations in the patient.

Optionally, the modifying of the acquisition parameter is executed further based on reference quality scores, being quality scores determined with respect to at least one previous physiological measurement of the patient which occurred at a previous date (i.e. different day). For example, if the same physiological examination in a previous date for a specific patient achieved a quality score of 7 out of 10, the acquisition parameters may be modified in order to achieve measurement of at least similar quality. For another patient - where the best quality achieved in the past was 5 out of 10 (e.g. because of her weight, body fat or anxiety) - it may be useless to attempt to achieve a higher level of measurement quality. The use of previous quality scores as reference enables the system to keep improving its quality of measurement, by using adaptable criterions.

Different acquisition parameters (and/or different criterions for necessity of modification) may be used for different patients. Optionally, the modifying of the acquisition parameter is executed further in response to a quality criterion selected for the patient by a medical professional (or by a computer, such as a processor of the physiological measurement system). For example, with different patients, different quality of images can even be achieved by doctors, let alone by the device. For example - optimal sound quality for hear exam and/or auscultation may depend on fat-concentrations in the patient. For example, patients with known heart condition may necessitate higher quality measurement in certain aspects, when compared to patient with no heart history.

It is noted that similar criterions and considerations to those discussed above with respect to stage 570 may be used for issuing an instruction at stage 560.

Fig. 7 illustrates optional stage 580 of method 500, according to examples of the presently disclosed subject matter. Stage 580 includes selecting, based on the quality scores, a proper part of the physiological data collected during the physiological measurement, and generating a physiological measurement preview based on the proper part for presenting by a tangible user interface (e.g. of a device operated by a medical practitioner). The physiological measurement preview may be part of the analysis source data (in which case stage 580 may be a part of stage 590), but this is not necessarily so (in which case stage 580 is executed after some or all of the instances of stage 540). In some cases, the physiological measurement preview can at least partially overlap to a given portion of the physiological data identified as diagnosis-enabling data. The physiological measurement preview may include, for example, one or few images, a short video clip, a thumbnail (static or dynamic), a short sound sample, and so on. It is noted that a proper part means a part but not all (A is a proper part of B if A is a part of B but B is not a part of A). Method 500 may also include presenting the physiological measurement preview, but this is not necessarily so. It is noted that stage 580 may optionally be executed on another system than the one executing other stages of method 500, such as a remote server.

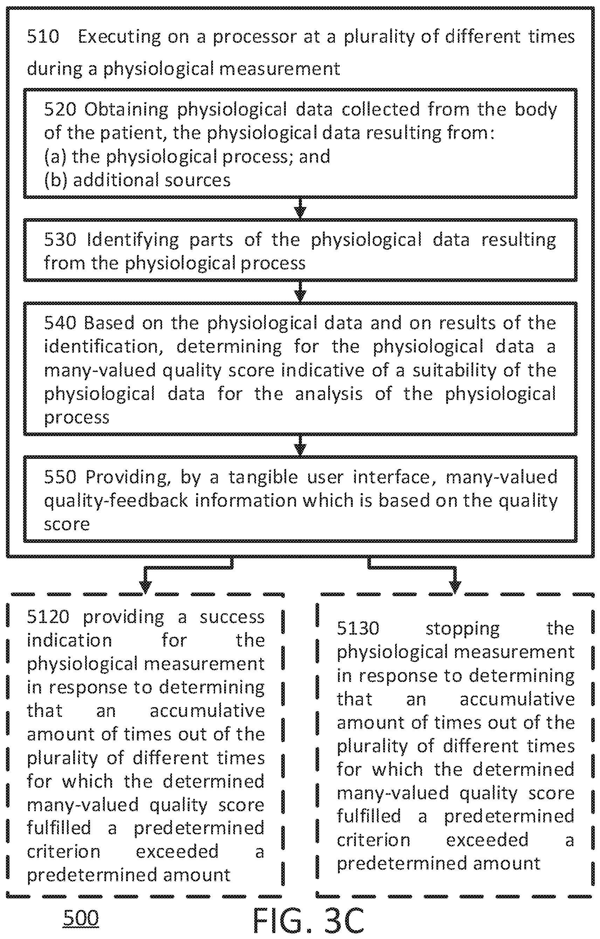

Fig. 3C is a flowchart illustrating an example of method 500, in accordance with the presently disclosed subject matter. In the example of Fig. 3C, use is made of several many-valued quality scores together in an accumulative fashion. Each of the optional stages 5120 and 5130 may be executed after stages 520, 530, 540 and 550 was executed multiple times.

Optional stage 5120 includes providing a success indication for the physiological measurement in response to determining that an accumulative amount of times out of the plurality of different times for which the determined many-valued quality score fulfilled a predetermined criterion exceeded a predetermined amount. Referring to the examples set forth with respect to the previous drawings, stage 5120 may be executed by processor 220 and/or UI 230.

Referring to processor 220, it is noted that optionally processor 220 may be configured to selectively provide a success indication for the physiological measurement in response to determining that an accumulative amount of times out of the plurality of different times for which the determined many-valued quality score fulfilled a predetermined criterion exceeded a predetermined amount.

The success indication may be provided to the user using a user interface (e.g. according to any of the UI examples provided above), to a processor of the measurement system and/or to any other system. The success indication may indicate that the physiological measurement was successful (i.e. that diagnosis-enabling data was obtained). Optionally, other indication may be used which indicates a failure of the physiological measurement.

For example, the success indication may be provided if the many-valued quality scores determined for different consecutive times fulfilled a predetermined criterion (e.g. exceeded a threshold) for a continuous time (e.g. the quality scores exceeded a score of 6 out of 10 for at least 5 consecutive seconds). For example, the success indication may be provided if the many-valued quality scores determined for different times fulfilled a predetermined criterion (e.g. exceeded a threshold) for a predetermined accumulative duration (e.g. the quality scores exceeded a score of 6 out of 10 for at least 15 not necessarily consecutive seconds). For example, the success indication may be provided if the many-valued quality scores determined for different times fulfilled a predetermined criterion (e.g. exceeded a threshold) for a predetermined number of consecutive, and optionally non-overlapping, durations (e.g. the quality scores exceeded a score of 6 out of 10 for at least 5 consecutive seconds at least 3 times).

Optional stage 5130 includes stopping the physiological measurement in response to determining that an accumulative amount of times out of the plurality of different times for which the determined many-valued quality score fulfilled a predetermined criterion exceeded a predetermined amount. Referring to the examples set forth with respect to the previous drawings, stage 5120 may be executed by processor 220 and/or UI 230. Referring to the examples set forth with respect to the previous drawings, stage 5130 may be executed by processor 220 (Referring to processor 220, it is noted that optionally processor 220 may be configured to stop the physiological measurement in response to determining that an accumulative amount of times out of the plurality of different times for which the determined many-valued quality score fulfilled a predetermined criterion exceeded a predetermined amount).

For example, the physiological measurement may be stopped if the many-valued quality scores determined for different consecutive times fulfilled a predetermined criterion (e.g. exceeded a threshold) for a continuous time, or if the many-valued quality scores determined for different times fulfilled a predetermined criterion (e.g. exceeded a threshold) for a predetermined accumulative duration, or if the many-valued quality scores determined for different times fulfilled a predetermined criterion (e.g. exceeded a threshold) for a predetermined number of consecutive, and optionally non-overlapping, durations.

It is noted that similar utilization of many-valued quality scores determined for physiological measurements may be useful even without necessarily identifying parts of the collected physiological data which originate from the physiological process which is to be analyzed.

For example, another method is hereby disclosed - a computer-implemented method for providing feedback indicative of a suitability of data collected during a physiological measurement for analysis of a physiological process of a body of a patient, which includes executing on a processor: (a) obtaining physiological data collected from the body of the patient at a plurality of different times during a physiological measurement, the physiological data resulting at least from the physiological process; (b) determining many-valued quality scores for the physiological data collected at the plurality of different times; and (c) selectively providing a success indication for the physiological measurement in response to determining that an accumulative amount of times, out of the plurality of different times, for which the determined many-valued quality score fulfilled a predetermined criterion exceeded a predetermined amount.

Stage (c) may be replaced (or joined) with a stage of selectively stopping the physiological measurement in response to determining that an accumulative amount of times, out of the plurality of different times, for which the determined many-valued quality score fulfilled a predetermined criterion exceeded a predetermined amount.

This method may include any variation discussed with respect to method 500, mutatis mutandis, but it does not necessitate identifying parts of the physiological data which result from the physiological process.

Likewise, a corresponding system may be disclosed, which does not necessitate such identification of parts of the physiological data which result from the physiological process. Such a system for physiological measurement of a physiological process of a body of a patient is hereby disclosed, including at least one physiological sensor operable to collect, at a plurality of different times during a physiological measurement, physiological data from the body of the patient, the physiological data resulting at least from the physiological process; and a processor operable to: (a) determine many-valued quality scores for the physiological data collected at the plurality of different times, and (b) to selectively provide a success indication for the physiological measurement in response to determining that an accumulative amount of times, out of the plurality of different times, for which the determined many-valued quality score fulfilled a predetermined criterion exceeded a predetermined amount.

The processor may be alternatively (or in addition) operable to: (a) determine many-valued quality scores for the physiological data collected at the plurality of different times, and (b) to selectively stop the physiological measurement in response to determining that an accumulative amount of times, out of the plurality of different times, for which the determined many-valued quality score fulfilled a predetermined criterion exceeded a predetermined amount.

This system may include any variation discussed with respect to system 200, mutatis mutandis, but the processor does not necessarily have to be able to identify parts of the physiological data which result from the physiological process.

Similarly, a non-transitory computer-readable medium for providing feedback indicative of a suitability of data collected during a physiological measurement for analysis of a physiological process of a body of a patient is hereby described, including instructions stored thereon, that when executed on a processor, perform on the processor at a plurality of different times during a physiological measurement the steps of: (a) obtaining physiological data collected from the body of the patient at a plurality of different times during a physiological measurement, the physiological data resulting at least from the physiological process; (b) determining many-valued quality scores for the physiological data collected at the plurality of different times; and (c) selectively providing a success indication for the physiological measurement in response to determining that an accumulative amount of times, out of the plurality of different times, for which the determined many-valued quality score fulfilled a predetermined criterion exceeded a predetermined amount.

Stage (c) may be replaced (or joined) with a stage of selectively stopping the physiological measurement in response to determining that an accumulative amount of times, out of the plurality of different times, for which the determined many-valued quality score fulfilled a predetermined criterion exceeded a predetermined amount.

This non-transitory computer-readable medium may include any variation discussed with respect to any non-transitory computer-readable medium discussed below, mutatis mutandis, but it does not necessitate identifying parts of the physiological data which result from the physiological process.

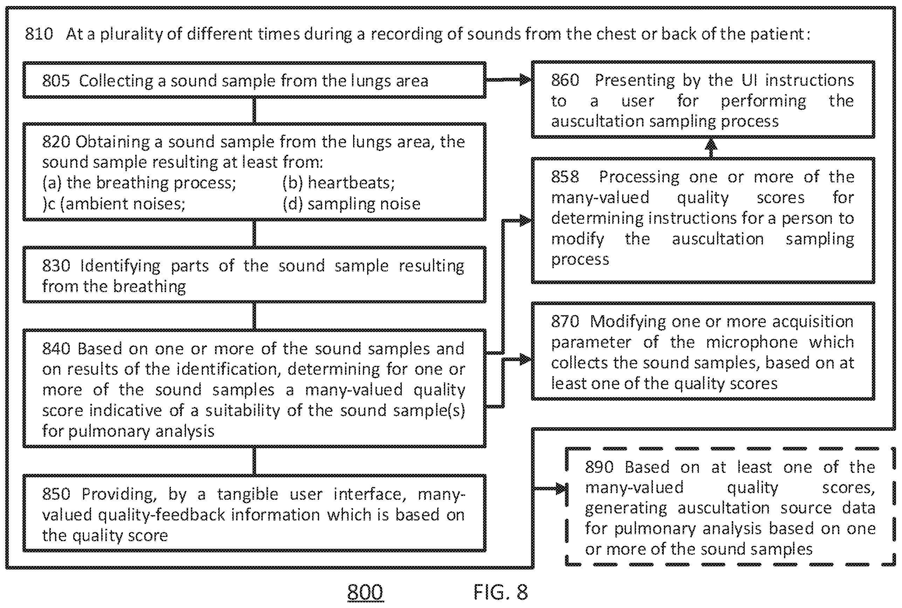

Fig. 8 is a flow chart illustrating an example of method 800, in accordance with the presently disclosed subject matter. Method 800 is a variation of method 500 for providing feedback indicative of a suitability of data collected during a pulmonary auscultation (i.e. recording of sounds from the lungs) for pulmonary analysis (i.e. relating to the lungs) of a patient. The pulmonary analysis may be aimed, for example, to diagnose/assess a condition of the lungs of the patient, of her ability to breath, of her breathing patterns, etc. Referring to the examples set forth with respect to the previous drawings, method 800 may be executed by system 200. If optional stage 890 is implemented, method 800 may be used for preparing auscultation source data for the pulmonary analysis of the patient (for diagnosing a medical condition relating to the lungs of the patient).

As method 800 is an implementation of method 500, details discussed above with respect to stage 510 may be applied, mutatis mutandis, to stage 810, details discussed above with respect to stage 520 may be applied, mutatis mutandis, to stage 820, and so on.

In method 800, the physiological data is sound samples recorded from the chest or back of the patient. For example, the sound samples may be collected by a portable handheld device which includes a microphone and a processor, such as the handheld medical devices produced by Tytocare LTD. Of Netanya, Israel.

Optional stage 805 includes collecting a sound sample from the lungs area.

Stage 820 includes obtaining a sound sample from the lungs area, the sound sample resulting from the breathing process, but optionally from other sources as well, such as heartbeats, ambient sounds, sampling noise, etc.

Stage 830 includes identifying parts of the sound sample resulting from the breathing. Ways in which the identification may be used to distinguish between sounds resulting from the breathing to sounds resulting from other sources were discussed above.

Stage 840 includes determining for one or more of the sound samples, based on one or more of the sound samples and on results of the identification, a many-valued quality score indicative of a suitability of the sound sample(s) for pulmonary analysis.

Stage 850 includes providing, by a tangible user interface, many-valued quality-feedback information which is based on at least one of the many-valued quality score.

Optional stage 890 includes generating auscultation source data for pulmonary analysis based on one or more of the sound samples and on at least one of the many-valued quality scores. The auscultation source data includes at least diagnosis-enabling data, enabling diagnosing a medical condition relating to the lungs of the patient.

Optional stage 860 includes presenting by the UI instructions to a user for performing the auscultation sampling process.

Optional stage 858 includes processing one or more of the many-valued quality scores for determining instructions for a person to modify the auscultation sampling process.

Optional stage 870 includes modifying one or more acquisition parameter of the microphone which collects the sound samples, based on at least one of the many-valued quality scores.

It is to be noted that the above method 800 may be implemented similarly also on other auscultation types (e.g. relating to the heart, bowel sounds, etc.), mutatis mutandis.

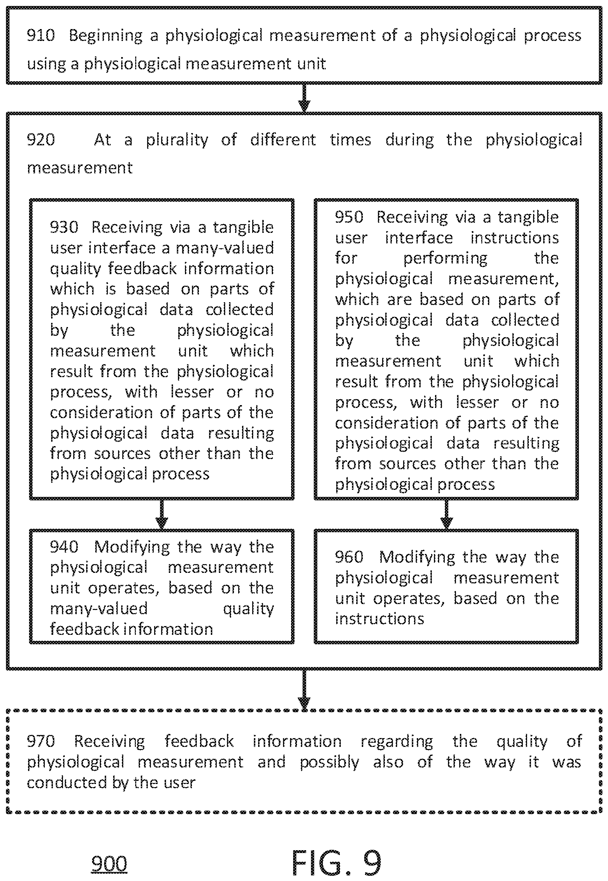

Fig. 9 is a flow chart illustrating an example of method 900, in accordance with the presently disclosed subject matter. Method 900 is a method which may optionally be implemented by the user which operates the physiological sensor/measurement device used for the physiological measurement of method 500 and/or by the user operating system 200. Any detail discussed above with respect to method 500 and/or to system 200 may be applicable, mutatis mutandis, to method 900, where applicable. Method 900 may be executed by the patient whose body is examined, or by another person (optionally a non-medical practitioner) in its vicinity.

Stage 910 includes beginning a physiological measurement of a physiological process using a physiological measurement unit. The physiological measurement unit may be a handheld portable device, or a larger device. The selection of the physiological measurement, the physiological process examined etc. may be done by the patient, by the operator, by the measurement unit, by another computer, or by a person at a remote location (e.g. a physician located in a remote center). Stage 910 may include setting parameters for the measurement, but this is not necessarily so.

At a plurality of different times during the physiological measurement (denoted 920), the user executes stage 930 followed by stage 940 and/or stage 950 followed by stage 960.

Stage 930 includes receiving via a tangible user interface a many-valued quality feedback information which is based on parts of physiological data collected by the physiological measurement unit which result from the physiological process, with lesser or no consideration of parts of the physiological data resulting from sources other than the physiological process.

The many-valued quality feedback information may be presented in one or more of different ways, e.g. as discussed above - as a digital numeral display, as a LED display, verbally, by non-speech audio, using vibration or other tactile information, and so on.

Stage 940 includes modifying the way the physiological measurement unit operates, based on the many-valued quality feedback information. The user can modify the measurement in different ways, such as: moving or readjusting the position/orientation of the measurement unit, changing its measurement parameters, readjusting her body position, breathing differently, replacing modules (e.g. otoscope speculum) of the measurement unit, or any other way discussed above.

Stage 950 includes receiving via a tangible user interface instructions for performing the physiological measurement. The instructions are based on parts of physiological data collected by the physiological measurement unit which result from the physiological process, optionally with lesser or no consideration of parts of the physiological data resulting from sources other than the physiological process. The instructions may be presented to the user in one or more of different ways, e.g. as discussed above - as a digital numeral display, as a LED display, verbally, by non-speech audio, using vibration or other tactile information, and so on.

Stage 960 includes modifying the way the physiological measurement unit operates, based on the instructions. The user can modify the measurement in different ways, as discussed above with respect to stage 940.

Optional stage 970 includes receiving feedback information regarding the quality of physiological measurement and possibly also of the way it was conducted by the user.

By implementing method 900, the user can improve the quality of physiological measurement to better suit to analysis of the physiological process, and also to learn better how to operate the measurement unit, thereby improving also the user's skills for subsequent physiological measurements (if any).

Also, by receiving feedback on the quality of the measurement and its suitability for analysis of the physiological process (during and/or after the conclusion of measurement), the user can generate a high-quality measurement which is suitable for analysis without the intervention of a trained expert.

This greatly reduces the chance that when the analysis source data (based on this physiological measurement) is ultimately analyzed (which may take longer times, e.g. hours or even days and weeks), the data would not be of sufficient quality for analysis (i.e. the analysis source data will not include diagnosis-enabling data).

Reverting to the examples of Figs. 1 and 2, it is noted that system 200 may implement any of the variations discussed above with respect to methods 500 and 800, mutatis mutandis. While the description of system 200 below is not limited to execution of method 500, it is hoped that it will be clearer after the detailed discussion of method 500.

System 200 is a system for preparing analysis source data for analysis of a physiological process of a body of a patient. System 200 includes at least one physiological sensor 210 which is operable to collect multiple times during a physiological measurement physiological data from the body of the patient, the physiological data resulting from: (a) the physiological process and from (b) additional sources.

Processor 220 is operable to execute at a plurality of different times during a physiological measurement: (a) identifying parts of the physiological data resulting from the physiological process; (b) based on the physiological data and on results of the identification, determining for the physiological data a many-valued quality score (i.e. more than 2 options) indicative of a suitability of the physiological data for the analysis of the physiological process; and (c) providing, by a tangible user interface, many-valued quality-feedback information which is based on the quality score.

Processor 220 is further operable to generate the analysis source data based on physiological data collected by the physiological sensor and on at least one of the many-valued quality scores.

While not necessarily so, the quality scores determined by processor 220 may be different than any value included in the analysis source data.

Optionally, processor 220 may be operable to identify the parts of the physiological data which result from the physiological process based on identification of effects of a plurality of different physiological processes on the physiological data.

System 2000 may include UI 230 which may be operable to present instructions to a user for performing the physiological measurement. In such a case, processor 220 may optionally determine (and possibly modify) the instructions based on at least one of the many-valued quality scores.

Optionally, processor 220 is configured to determine at least one of the many-valued quality scores further based on parameters of an analysis procedure selected out of a predefined finite plurality of analysis procedures for analyzing the physiological data.

For example, system 200 may be used at one time for auscultation of the heart, and on another time for auscultation of the lungs. Processor 220 in such case can be configured to determine at least one of the many-valued quality scores based on the type of auscultation. The selection of what is the physiological process and/or what sort of analysis the collected physiological data will be used for may be made by a user, automatically, or by a remote system. It is noted that this selection may change in different times.

Optionally, processor 220 is further operable to select, based on the quality scores, a proper part of the physiological data collected during the physiological measurement, and to generate a physiological measurement preview based on the proper part for presenting by a tangible user interface.

It is noted that processor 220 may determine different many-valued quality scores for a plurality of different successful physiological measurements (examples for the meaning of the term "successful" are discussed above with respect to method 500).

Optionally, a physiological sensor 210 out of the at least one physiological sensor 210 may utilize for at least one of the measurements an acquisition parameter that is based on at least one of the quality scores. This at least one of the quality scores is a many-valued quality score which was previously determined for physiological data collected by the physiological sensor 210 during the same physiological measurement.

Optionally, the aforementioned acquisition parameter is determined further in response to a quality criterion selected for the patient by a medical professional.

Optionally, the aforementioned acquisition parameter is determined further in response to a medical condition of the patient.

Optionally, the aforementioned acquisition parameter is determined further in response to quality scores determined with respect to at least one previous physiological measurement which occurred at a previous time/date.

Optionally, the many-valued quality scores are indicative of a degree in which the patient follows instructions for physical activities.

Optionally, processor 220 may be configured to determine the many-valued quality scores based on a selection of a scoring scheme out of a plurality of predefined scoring schemes, wherein each scoring scheme is associated with an analysis process for the physiological process.

Optionally, processor 220 may be configured to compress, for the analysis source data, different parts of the physiological data based on different many-valued quality scores determined for the different parts. Optionally, communication module 240 may be operable to transmitting the analysis source data, which includes the compressed physiological data, to an external system.

In some cases, parts of the physiological data which were assigned a low-quality score may be compressed using higher compression level (and/or lower preservation rate of compression) with respect to parts which received higher quality scores. It is noted that some parts may be omitted from the analysis source data altogether (e.g. if their many-valued quality score indicates irrelevancy or unsuitability for analysis - e.g. because the signal is of inferior quality, because it does not include information of a relevant body part).

Optionally, processor 220 may be configured to determine the many-valued quality scores based on criteria determined by an expert at a remote location. Optionally, the criteria may be determined by the expert (e.g. physician, technician) during the physiological measurement, possibly based on data previously collected at an earlier time of the physiological measurement. For example, the expert may indicate points of interest (POI), such as specific locations on the body, specific range of acoustic measurement data, and so on.

It is noted that system 200 may optionally include one or more non-physiological sensor (e.g. IMU, microphone, or other examples discussed above with respect non-physiological sensors in method 500). Processor 220, in such a case, may be configured to determine the many-valued quality score for at least one physiological data further based on data collected by the at least one non-physiological sensor.

It is noted that the determining of the many-valued quality scores by processor 220 may also be based only on the physiological data and on results of the identification, without any additional data. It is noted that the determining of the many-valued quality scores by processor 220 may also be implemented based on the physiological data and on results of the identification, without any additional measurement data (but possibly using some other forms of data such as clock data, etc.).

Any reference in the specification to method 500 or 800 should be applied mutatis mutandis to a system capable of executing the respective method and should be applied mutatis mutandis to a non-transitory computer readable medium that stores instructions that once executed by a computer result in the execution of the method.

For example, a non-transitory computer-readable medium for providing feedback indicative of a suitability of data collected during a physiological measurement for is disclosed. The non-transitory computer-readable medium including instructions stored thereon, that when executed on a processor, perform the steps of:
a. executing at a plurality of different times during a physiological measurement:
   i. obtaining physiological data collected from the body of the patient, the physiological data resulting from: (a) the physiological process and from (b) additional sources;
   *ii.* identifying parts of the physiological data resulting from the physiological process;
   iii. based on the physiological data and on results of the identification, determining for the physiological data a many-valued quality score indicative of a suitability of the physiological data for the analysis of the physiological process; and
   iv. providing, by a tangible user interface, many-valued quality-feedback information which is based on the quality score; and

Optionally, the non-transitory computer-readable medium may further include instructions stored thereon, that when executed on the processor, perform: based on at least one of the many-valued quality scores, generating the analysis source data based on the physiological data obtained at at least one of the plurality of different times.

Optionally, the quality score may be different than any value included in the analysis source data.

Optionally, the identifying may be based on identification of effects of a plurality of different physiological processes on the physiological data.

Optionally, the plurality of different times may include at least a first time and a second time which is later than the first time, and the obtaining of the physiological data at the second time is affected by changes of the physiological measurement by the user as a result from providing by the tangible user interface of the many-valued quality feedback information resulting from many-valued determined for physiological data obtained at the first time.

Optionally, the physiological data may be collected by a physiological measurement device, and the suitability of the physiological data changes as a result of changes in operating of the physiological measurement device by a user which perceives the quality feedback information presented by the tangible user interface.

Optionally, the non-transitory computer-readable medium may further include instructions stored thereon, that when executed on the processor, perform presenting by the tangible user interface instructions to a user for performing the physiological measurement.

Optionally, the determining of the many-valued quality score may be further based on one or more parameters of an analysis procedure selected out of a predefined finite plurality of analysis procedures for analyzing the physiological data.

Optionally, the obtaining, identifying and determining may be executed by a portable handheld physiological monitoring device, wherein the obtaining includes measuring the physiological measurement by at least one physiological sensor of the portable handheld physiological monitoring device.

Optionally, the non-transitory computer-readable medium may further include instructions stored thereon, that when executed on the processor, perform:
a. selecting, based on the quality scores, a proper part of the physiological data collected during the physiological measurement, and
b. generating a physiological measurement preview based on the proper part for presenting by a tangible user interface.

Optionally, the non-transitory computer-readable medium may further include instructions stored thereon, that when executed on the processor, perform modifying an acquisition parameter of a physiological sensor which collects at least a part of the measurement data based on at least one of the quality scores.

Optionally, the modifying of the acquisition parameter may be executed further in response to a quality criterion selected for the patient by a medical professional.

Optionally, the modifying of the acquisition parameter may be executed further in response to a medical condition of the patient.

Optionally, the modifying of the acquisition parameter may be executed further in response to quality scores determined with respect to at least one previous physiological measurement which occurred at a previous date.

Optionally, the many-valued quality scores may be indicative of a degree in which the patient follows instructions for physical activities.

Optionally, the determining of the many-valued quality score may be based on a selection of a scoring scheme out of a plurality of predefined scoring schemes, wherein each scoring scheme is associated with an analysis process for the physiological process.

Optionally, the generating of the analysis source data may include compressing different parts of the physiological data based on different many-valued quality scores determined for the different parts.

Optionally, the determining of the many-valued quality score for at least one physiological data may further be based on data collected by non-physiological sensor of a physiological measurement system which collected the physiological data.

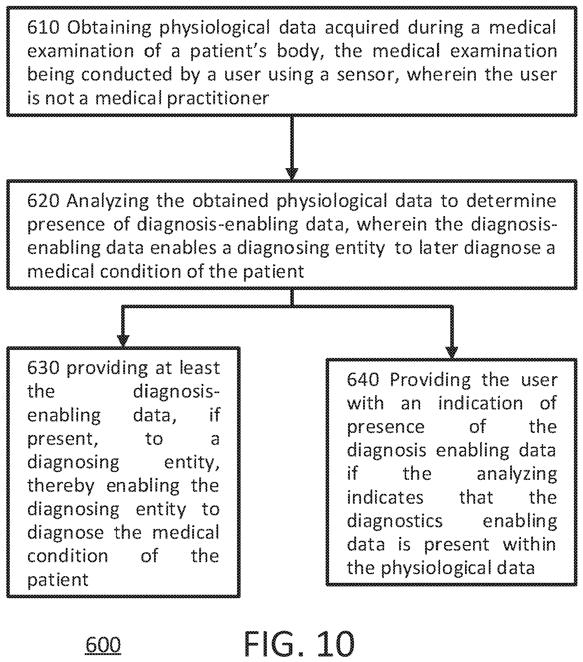

Attention is now drawn to Fig. 10, a flowchart illustrating an example of a method 600, in accordance with the presently disclosed subject matter. Method 600 is a method for providing feedback indicative of presence/absence of diagnosis-enabling data within physiological data collected from a body of a patient. Referring to the examples set forth with respect to the previous drawings, method 600 may be executed by system 200. Any variation, combination or optional implementation which is discussed with respect to system 200 may be implemented, mutatis mutandis, also with respect to method 600. Any variation, combination or optional implementation which is discussed with respect to method 600 may be implemented, mutatis mutandis, also with respect to system 200. Method 600 can be executed by processor 200 in addition to, or instead of, method 500. In some cases, method 600 can be executed as part of method 600.

Method 600 is executed during a physiological measurement, and includes executing on a processor, once, or at a plurality of different times during the physiological measurement, the stages 610 and 620, and optionally one or both stages 630 and 640. It is to be noted that any one, or more (and optionally all), of the stages 610, 620, 630 and 640 can be executed by processor 220.

Stage 610 includes obtaining physiological data collected from the body of the patient during a medical examination of the patient. The physiological data can be data obtained by an instantaneous measurement, or data obtained non-instantaneously but during a certain period of time. In some cases, the physiological data can be data resulting from: (a) a physiological process and from (b) additional sources. As discussed with respect to system 200, the physiological data collected at stage 610 may be collected by one or more sensors. The medical examination can be conducted by a user using the one or more sensors. The user conducting the medical examination is not necessarily a medical practitioner having formal medical training, and in some cases, the user is not a medical practitioner.

Stage 620 includes analyzing the obtained physiological data to determine presence of diagnosis-enabling data. Diagnosis-enabling data is data that enables a diagnosing entity (such as a medical practitioner, a computerized diagnosis system, etc.) to diagnose a medical condition of the patient. It is to be noted that in order for a diagnosing entity to be able to diagnose based on the diagnosis-enabling data, the diagnosis-enabling data is required to be of a certain minimal quality, that enables such diagnosis.

It is to be noted that in some cases, the diagnosing entity performs the diagnosis based on the diagnosis-enabling data at a later time, after execution of method 600. Therefore, it is desirable to verify that the obtained physiological data comprises diagnosis-enabling data so that once the diagnosing entity performs the diagnosis, it will have access to diagnosis-enabling data. Otherwise, the medical examination of the patient (during which the physiological data is collected) will have to be repeated in order to obtain new or additional physiological data from the patient's body. This naturally requires the availability of the patient, and optionally, if the patient is not operating the sensor himself, also of a user operating the sensor. In addition to the need to repeat the medical examination, in case the physiological data does not comprise diagnosis-enabling data, the diagnosing entity's resources are wasted, as it will attempt to diagnose a medical condition of the patient based on data that does not comprise diagnosis-enabling data, and therefor - it will fail, or provide a poor or an erroneous diagnosis.

It is to be noted that the determination whether the physiological data includes diagnosis-enabling data can be performed utilizing any one or more of the substages of stage 530, i.e. one or more of stages 531 through 538 detailed above. In some cases, additional/alternative methods can be used to determine presence of diagnosis-enabling data within the obtained physiological data. In more specific cases, the determination can be made based on a many-valued quality score determined based on the results of performance of the one or more substages of stage 530, as detailed above with respect to stage 540. In such cases, the calculated score may be required to exceed a certain threshold, that can optionally depend on the specific medical examination conducted on the patient, and/or on the specific patient's characteristics, as detailed above, etc. In some cases, the analysis for determining presence of diagnosis-enabling data is performed on those parts of the physiological data identified as originating from the physiological process.

In a specific example, where the physiological data is audio data, the determination whether the physiological data includes diagnosis-enabling data can be performed as follows: the physiological data can be converted to the frequency domain. Then, it is weighted by the frequency, e.g. using an equal-loudness curve. The energy of the weighted signal can then be calculated and converted to log-scale, after which it can be rescaled to match the number of options for values of the many-valued quality score.

Stage 630 includes providing (e.g. by the processor 220) at least the diagnosis-enabling data to the diagnosing entity, thereby enabling the diagnosing entity to diagnose the medical condition of the patient, If the analysis shows that diagnosis-enabling data exists within the obtained physiological data. Providing the diagnosis-enabling data to the diagnosing entity can include transmitting the diagnosis-enabling data, via a network interface (whether wired or wireless), to a separate device (e.g. a computerized workstation, a smartphone, a tablet, etc.), other than system 200, the separate device operated by a medical practitioner.

In some cases, the data provided at stage 630 includes the diagnosis-enabling data, and additional data. In such cases, an indication of the location of the diagnosis-enabling data within the data provided at stage 630 can be also provided. For example, the entirety of the physiological data obtained at stage 610, including the diagnosis-enabling data and additional data that is not diagnosis-enabling (e.g. data having a poor quality), can be provided at stage 630, and an indication about the location (or a plurality of locations) of the diagnosis-enabling data within the obtained physiological data can be provided so that the diagnosing entity can locate the diagnosis-enabling data within the provided data (e.g. in order to save seek times of seeking the data for the diagnosis-enabling data).

Stage 640 includes providing (e.g. by the processor 220) a user operating the system 200 (e.g. the patient or another non-medical practitioner such as a family member of the patient), e.g. using a user interface 230, with an indication of presence/absence of diagnosis-enabling data within the obtained physiological data, if and when the analysis of stage 620 shows that the physiological data comprises diagnosis-enabling data. In some cases, the indication can be a V mark if diagnosis-enabling data (or a sufficient amount thereof) is present and an X mark of diagnosis-enabling data is absent within the obtained physiological data.

As indicated herein, in some cases, the user interface 230 can be part of a handheld medical device operated by the user (e.g. a display, a speaker, one or more vibrating elements, a group of LEDs, etc.). Additionally, or alternatively, the user interface 230 can be external to a handheld medical device operated by the user, such as an external display, an output means of a smartphone (a smartphone display, speaker, vibrating elements, etc.) or another computer (where the information can be provided on a user interface of that computer) and so on (in such cases, the indication of presence/absence of diagnosis-enabling data within the obtained physiological data can be provided to such external user interface via a wired/wireless connection).

It is noted that UI 230 may optionally be used to provide additional information to a user of system 200, whether originating from processor 220 or not. For example, UI 230 may optionally additionally provide instructions for how to change measurement for improving quality of the measurement, for indicating an end of measurement (or measurement part, e.g. moving to another location on the body to continue the measurement) and so on. Such additional information may optionally be provided by UI 230 during the examination, and not only after it concludes. However, it is not necessary that any information (whether the many-valued quality-feedback information or any other information provided by UI 230) would be provided at all times (or at any specific time) throughout the examination.

In some cases, the physiological data is obtained and analyzed in real-time (e.g. immediately, or substantially immediately (e.g. within up to five seconds, however this is a non-limiting example), after the physiological data is acquired by the at least one physiological sensor 210). In some cases, the indication of presence of diagnosis-enabling data within the obtained physiological data is also provided in real-time (e.g. immediately, or substantially immediately (e.g. within up to five seconds, however this is a non-limiting example), after determining that diagnosis-enabling data exists within the obtained physiological data), thereby enabling the user operating the system 200 to determine when to stop performance of the physiological examination of the patient. It is to be noted that in some cases the indication of presence of diagnosis-enabling data within the obtained physiological data can be provided at a certain point-in-time after the processor 220 determines that diagnosis-enabling data exists within the obtained physiological data (e.g. up to a few seconds or a few minutes later).

In those cases where the physiological data is obtained and analyzed in real-time (e.g. immediately, or substantially immediately, after the physiological data is acquired by the at least one physiological sensor 210), the processor 220 can be configured to provide the user, upon determining that the obtained physiological data comprises diagnosis-enabling data, with an instruction to spatially reposition the sensor with respect to the patient's body in accordance with the medical examination or in accordance with a subsequent medical examination defined by a pre-defined check plan (e.g. defining a certain sequence of one or more medical examinations) of the patient.

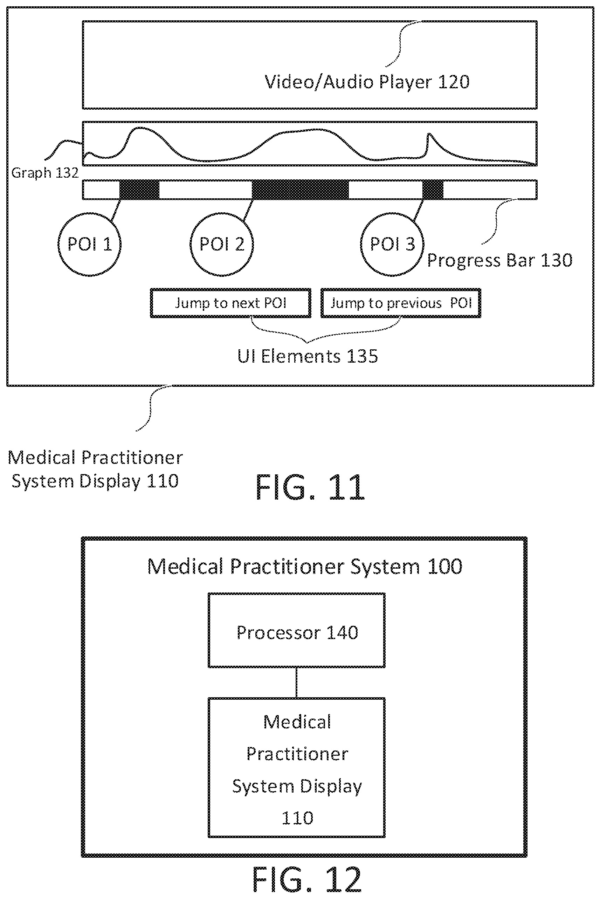

Attention is drawn to Fig. 11, showing an illustration of a user interface shown on a display of a medical practitioner system and enabling navigation to Points of Interest (POIs) within physiological data obtained during a non-instantaneous physiological measurement, in accordance with the presently disclosed subject matter.

As indicated herein (e.g. with respect to stage 590), in some cases the physiological data provided to a remote (e.g. the physiological measurement can be conducted at a first geographical location and transmitted to a second geographical location of the medical practitioner, the second geographical location can be remote from the first geographical location, e.g. a different street/city/province/country/etc.) medical practitioner (e.g. a physician, a technician, or any other entity that is allowed to view the acquired physiological data, e.g. for diagnosis purposes) may be accompanied by metadata indicative of specific portions of the physiological data identified as diagnosis-enabling data (e.g. as they are more suitable for analysis for the purpose of providing a diagnosis, as they are, for example, of a higher quality than other portions of the physiological data not identified as diagnosis-enabling data). Such metadata can be used in order to provide the medical practitioner with the ability to navigate to those portions of the physiological data that are identified as diagnosis-enabling data. It is to be noted in this respect that the need to navigate the physiological data arises in those cases where the physiological data is obtained during a non-instantaneous physiological measurement, i.e. a physiological measurement acquired over a certain non-instantaneous period of time, and not at a specific instantaneous point in time. In some cases, the period of time can be a period of more than ten seconds, more than one minute, etc.

It is to be noted that in some cases the physiological data (comprised in one or more files, such as video and/or audio files) can include data acquired over a long period of time, where only a certain smaller amount of the data is diagnosis-enabling data. Using the metadata indicative of specific portions of the physiological data identified as diagnosis-enabling data enables saving time of a medical practitioner analyzing the data, by enabling him to relate only to those portions of the physiological data identified as diagnosis-enabling data. In some cases, the portions of the physiological data identified as diagnosis-enabling data can be less than 50% of the physiological data, or even less. Therefore, the time saving of the medical practitioner is substantial.

In the illustrated example, an exemplary medical practitioner system display 110 is shown. The display 110 can be a computer display or any other display (including a display of a smartphone, a tablet computer, or any other device operated by a medical practitioner).

The physiological data can be an audio stream or a video stream. In such cases, a video/audio player 120 may be displayed on the display 110, along with a progress bar 130 associated with the video/audio stream being the physiological data. The progress bar 130 enables navigating the video/audio stream to specific points in time of the video/audio stream. The metadata indicative of specific portions of the physiological data identified as diagnosis-enabling data can be used to provide respective indications over the progress bar 130.

In the illustrated example, three portions of the physiological data are identified as diagnosis-enabling data. Such portions can be marked by assigning a certain color to those sections of the progress bar 130 associated with the portions of the physiological data identified as diagnosis-enabling data. Additionally, or alternatively, flags may be provided on the display 110, each pointing at the respective portion of the progress bar associated with the physiological data identified as diagnosis-enabling data of the progress bar 130 (e.g. POI 1, POI 2, POI 3 shown in the illustration). In some cases, the flags can point to a start location of the respective portions, and optionally also to an end location of the respective portions within the progress bar 130.

In addition to, or as an alternative for, the marking of specific portions of the progress bar, a graph (e.g. graph 132 shown in the figure) indicative of a relative quality of the physiological data over time can be displayed on the display 110. In such cases, the metadata associated with the physiological data can include a plurality of quality scores (optionally many-valued quality scores) calculated at a plurality of different times during the physiological measurement (during which the physiological data is obtained). Each quality score can be indicative of a suitability of the physiological data in the corresponding point-in-time for diagnosis by a medical practitioner. The graph 132 can be generated based on the plurality of quality scores, and presenting it to the medical practitioner can enable the medical practitioner to navigate to those parts of the physiological data that include diagnosis-enabling data (or, at least, having a relatively higher quality than other parts of the physiological data).

In some cases, various navigation User Interface (UI) elements 135 can be displayed on the display, in addition to the progress bar 130. Such navigation UI elements 135 can enable a user of the system to quickly navigate between portions of the physiological data identified as diagnosis-enabling data (e.g. POI 1, POI 2, POI 3 shown in the illustration). In some cases, the navigation UI elements 135 can be buttons enabling jumping to a next, or a previous POI (being a portion portions of the physiological data identified as diagnosis-enabling data), from any current location within the physiological data.

In some cases, the medical practitioner system (not shown) can enable the medical practitioner to communicate with another medical practitioner (e.g. an expert of a certain type of disease, etc.) for obtaining information therefrom. For this purpose, the user interface provided to the medical practitioner on the display 110 can enable the medical practitioner to provide an indication of one or more specific areas-of-interest (being certain partial portions of the physiological data) identified by the medical practitioner, and the medical practitioner system can be configured to send the physiological data, along with metadata indicative of the areas-of-interest identified by the medical practitioner, to a second medical practitioner system of a second medical practitioner. This can enable the second medical practitioner system to mark the specific areas-of-interest marked by the medical practitioner on the second medical practitioner system's display (e.g. on a progress bar shown therein) for enabling the second medical practitioner to navigate to the marked area-of-interest within the physiological data (in a similar manner to navigating, by the medical practitioner, to those parts of the physiological data that include diagnosis-enabling data).

In some cases, the medical practitioner system (not shown) can enable the medical practitioner to store various metadata generated thereby, along with an indication that such metadata was generated by the medical practitioner by which it was generated. Such metadata can be stored for example on an Electronic Health Record (EHR) associated with the patient from which the physiological data originates. In some cases, the user interface provided to the medical practitioner on the display 110 can enable the medical practitioner to provide an indication of one or more specific areas-of-interest (being certain partial portions of the physiological data) identified by the medical practitioner, and the medical practitioner system can be configured to store such indication as metadata associated with the physiological data being displayed to the medical practitioner.

Fig. 12 shows a functional block diagram illustrating an exemplary medical practitioner system, in accordance with the presently disclosed subject matter. Medical practitioner system 100 can comprise a processor 140, and a medical practitioner system display 110. Medical practitioner system 100 can be a workstation, a smartphone, a tablet computer, or any other device operated by a medical practitioner, and having a display 110. The processor 140 can be configured to control relevant medical practitioner system 100 resources and to enable operations related to the medical practitioner system 100.

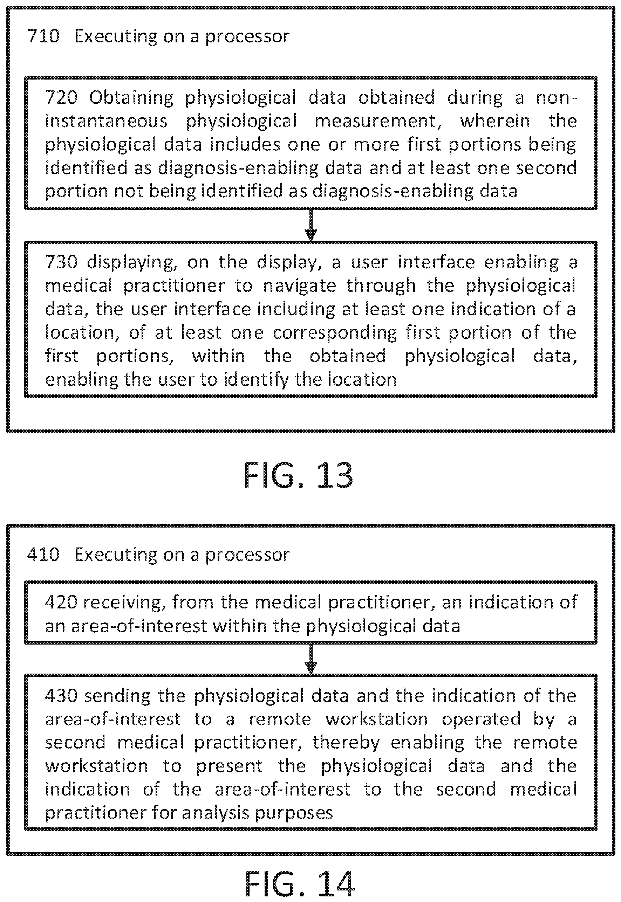

Attention is drawn to Fig. 13, showing a flowchart illustrating one example of a sequence of operations carried out for enabling navigation to Points/Areas of Interest (POls) within physiological data obtained during a non-instantaneous physiological measurement, in accordance with the presently disclosed subject matter.

Block 710 is a method for displaying, on the display 110, a user interface enabling navigation to Points/Areas of Interest (POIs) within physiological data obtained during a non-instantaneous physiological measurement. The method of block 710 can be executed by processor 140, executing steps 720 and 730.

Step 720 includes obtaining physiological data obtained during a non-instantaneous physiological measurement, wherein the physiological data includes one or more first portions being identified as diagnosis-enabling data and at least one second portion not being identified as diagnosis-enabling data.

Step 730 includes displaying, on the display 110, a user interface enabling a medical practitioner to navigate through the physiological data, the user interface including at least one indication of a location, of at least one corresponding first portion of the first portions, within the obtained physiological data, enabling the user to identify the location.

A visual illustration and further explanation about the user interface is provided with reference to Fig. 11.

Turning to Fig. 14, there is shown a flowchart illustrating one example of a sequence of operations carried out for providing a second medical practitioner with physiological data and an indication of areas-of-interest for consideration, in accordance with the presently disclosed subject matter.

Block 410 is a method for providing a second medical practitioner with physiological data and an indication of areas-of-interest, within the physiological data, for consideration. The method of block 410 can be executed by processor 140, executing steps 420 and 430.

Step 420 includes receiving, from the medical practitioner, an indication of an area-of-interest within the physiological data.

Step 430 includes sending the physiological data and the indication of the area-of-interest to a remote workstation operated by a second medical practitioner, thereby enabling the remote workstation to present the physiological data and the indication of the area-of-interest to the second medical practitioner for analysis purposes. In some cases, POIs are displayed in a manner that enables determining their origins (e.g. POIs generated by the system 200 are displayed in a first color, POIs generated by a first medical practitioner are displayed in a second color, other than the first color, POIs generated by a third medical practitioner are displayed in a third color, other than the first and second colors, etc.). In some cases, the POIs can be displayed in a manner that enables showing POIs generated by one or more selected origins (e.g. the system 200, one or more selected medical practitioners), so that some POIs associated with the physiological data are displayed to the second medical practitioner, whereas some POIs associated with the physiological data are not displayed to the second medical practitioner.

Further explanation about the method of block 410 is provided with reference to Fig. 11.

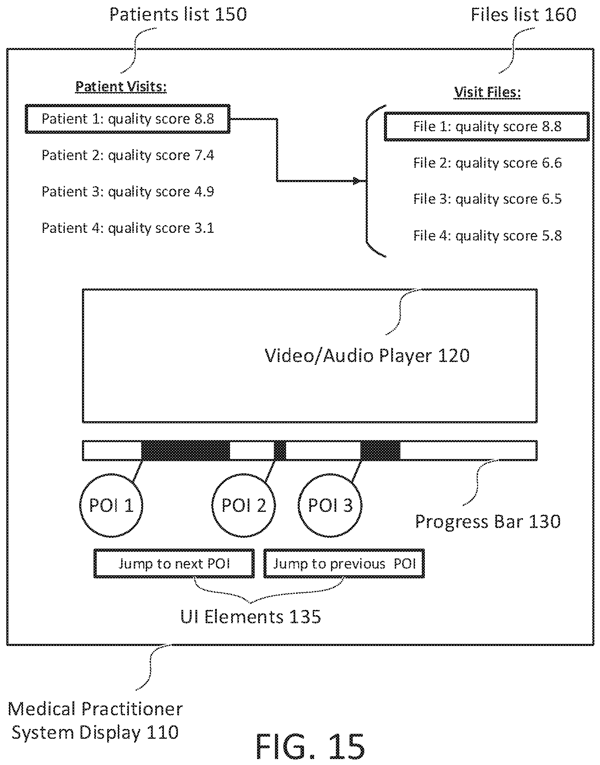

Turning to Fig. 15, there is shown an illustration of another user interface shown on a display of a medical practitioner system and enabling a medical practitioner to manage virtual visits of a plurality of patients, in accordance with the presently disclosed subject matter.

In accordance with the presently disclosed subject matter, one or more medical practitioners, can each be provided with one or more virtual patient visits. A virtual patient visit includes providing the medical practitioner with information about the patient, including physiological data acquired during a physiological measurement. The physiological measurement can be performed utilizing system 200. As indicated herein, the diagnosing entity (e.g. the medical practitioner) can be located remotely from the patient (e.g. the physiological measurement can be conducted at a first geographical location and transmitted to a second geographical location of the medical practitioner, the second geographical location can be remote from the first geographical location, e.g. a different street/city/province/country/etc.). In case two or more medical practitioners exist, the medical practitioners (and the respective medical practitioner system 100 operated thereby) can be located remotely from one another (e.g. in a different room/street/city/province/country/etc.).

In the illustrated example, an exemplary medical practitioner system display 110 is shown. The display 110 can be a computer display or any other display (including a display of a smartphone, a tablet computer, or any other device operated by a medical practitioner).

The medical practitioner system 100 can generate a user interface on the medical practitioner system display 110, and the user interface can include a patients list 150, which is a queued list of patient visits. Each patient visit is associated with a corresponding patient requesting medical diagnosis services, based on physiological data acquired during physiological measurements taken from the body of the corresponding patient, e.g. using system 200.

In some cases, the patients list 150 can be ordered, optionally in a descending order of quality scores determined for each patient visit. The quality score for the patient visit can be a maximal quality score of one or more files comprising physiological data acquired from the patient's body during the physiological measurements acquired for the patient visit (e.g. using system 200).

Upon a selection of a certain patient visit, from the patients list 150, the user interface can provide the medical practitioner operating the medical practitioner system 100, with a files list 160 comprising one or more files, each file comprising physiological data acquired from the patient's body during the physiological measurements acquired for the patient visit (e.g. using system 200).

In some cases, the files list 160 can be ordered, optionally in a descending order of quality scores determined for each file. As indicated herein, the quality score (that can optionally be many-valued) is indicative of a suitability of the physiological data (within the file) for the analysis of the specific physiological process - and it can be calculated based at least on identification of parts of the physiological data which result from the corresponding physiological process being measured. As quality scores are calculated at a plurality of times during a physiological measurement, the quality score of each file can be the maximal quality score calculated during the measurement during which the physiological data, for which the quality score was calculated, was acquired.

Upon selection of a specific file from the files list by the medical practitioner, a video/audio player 120 may be displayed on the display 110, along with a progress bar 130 associated with the video/audio stream being the physiological data comprised within the specific selected file (that can be an audio stream or a video stream). The user interface shown in Fig. 15 can enable the medical practitioner to perform any action detailed herein with reference to Fig. 11, for the selected file. This includes enabling the medical practitioner to navigate the physiological data using user interface markings/graphs determined in accordance with metadata accompanying the physiological data comprised within the selected file. This further includes enabling the medical practitioner to communicate with another medical practitioner (e.g. an expert of a certain type of disease, etc.) for obtaining information therefrom, while providing the other medical practitioner with an indication of one or more specific areas-of-interest to investigate.

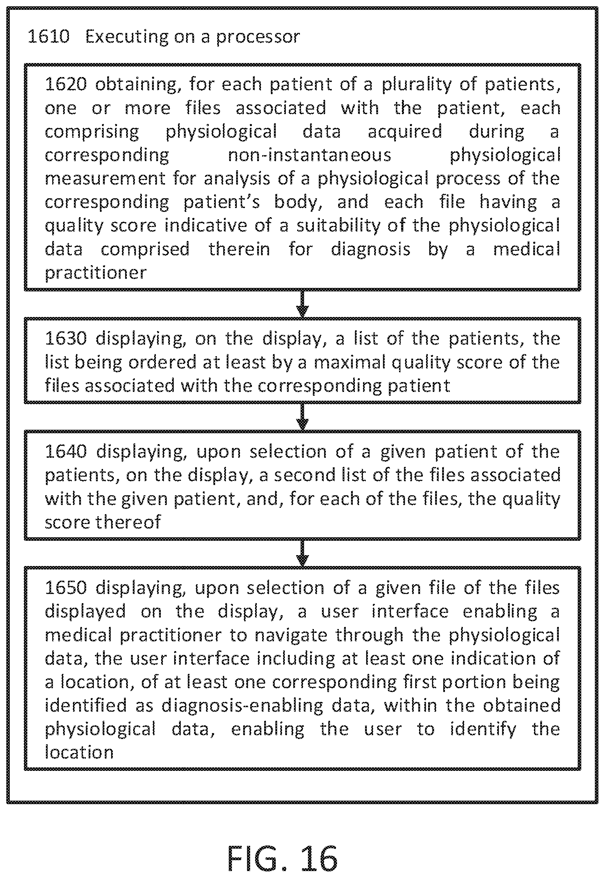

Turning to Fig. 16, there is shown a flowchart illustrating one example of a sequence of operations carried out for enabling a medical practitioner to manage virtual visits of a plurality of patients, in accordance with the presently disclosed subject matter.

Block 161 is a method for enabling a medical practitioner to manage virtual visits of a plurality of patients. The method of block 1610 can be executed by processor 140, executing steps 1620, 1630, 1640 and 1650.

Step 1620 includes obtaining, for each patient of a plurality of patients, one or more files associated with the patient, each comprising physiological data acquired during a corresponding non-instantaneous physiological measurement for analysis of a physiological process of the corresponding patient's body, and each file having a quality score indicative of a suitability of the physiological data comprised therein for diagnosis by a medical practitioner.

Step 1630 includes displaying, on the display 110, a patients list 150 being a list of the patents, the list being ordered at least by a maximal quality score of the files associated with the corresponding patient.

Step 1640 includes displaying, upon selection of a given patient of the patients list 150, on the display 110, a second list of the files (files list 160) associated with the given patient, and, for each of the files, the quality score thereof.

Step 1650 includes displaying, upon selection of a given file of the files list 160 displayed on the display 110, a user interface enabling a medical practitioner to navigate through the physiological data, the user interface including at least one indication of a location, of at least one corresponding first portion being identified as diagnosis-enabling data, within the obtained physiological data, enabling the user to identify the location.

Further explanation about the method of block 1610 is provided with reference to Fig. 15. It is to be further noted that method of block 1610 can further enable a medical practitioner to perform the method of block 410.

The presently disclosed subject matter may also be implemented by a computer program for running on a computer system, at least including code parts for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, a method, an implementation, an executable application, an applet, a servlet, a source code, code, a shared library/dynamic load library and/or other sequence of instructions designed for execution on a computer system.

The computer program may be stored internally on a non-transitory computer readable medium. All or some of the computer program may be provided on computer readable media permanently, removably or remotely coupled to an information processing system. The computer readable media may include, for example and without limitation, any number of the following: magnetic storage media including disk and tape storage media; optical storage media such as compact disk media (e.g., CD-ROM, CD-R, etc.) and digital video disk storage media; nonvolatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the presently disclosed subject matter has been described with reference to specific examples of embodiments. It will, however, be evident that various modifications and changes may be made therein without departing from the broader scope of the presently disclosed subject matter as set forth in the appended claims.

The connections as discussed herein may be any type of connection suitable to transfer signals from or to the respective nodes, units or devices, for example via intermediate devices. Accordingly, unless implied or stated otherwise, the connections may for example be direct connections or indirect connections. The connections may be illustrated or described in reference to being a single connection, a plurality of connections, unidirectional connections, or bidirectional connections. However, different embodiments may vary the implementation of the connections. For example, separate unidirectional connections may be used rather than bidirectional connections and vice versa. Also, plurality of connections may be replaced with a single connection that transfers multiple signals serially or in a time multiplexed manner. Likewise, single connections carrying multiple signals may be separated out into various different connections carrying subsets of these signals. Therefore, many options exist for transferring signals.

However, other modifications, variations and alternatives are also possible. The specifications and drawings are, accordingly, to be regarded in an illustrative rather than in a restrictive sense.

It will be appreciated that the embodiments described above are cited by way of example, and various features thereof and combinations of these features can be varied and modified.

While various embodiments have been shown, and described, it will be understood that there is no intent to limit the presently disclosed subject matter by such disclosure, but rather, it is intended to cover all modifications and alternate constructions falling within the scope of the presently disclosed subject matter, as defined in the appended claims.

## Claims

1. A system (100) comprising a processor (140) and a display (110), wherein the processor (140) is configured to:
obtain physiological data obtained during a non-instantaneous physiological measurement of a patient, wherein the physiological data includes audio data or video data;
analyze the physiological data to identify (a) one or more first portions of the physiological data that include diagnosis-enabling data, enabling a diagnosing entity to later diagnose a medical condition of the patient, wherein the first portions having a many valued quality score exceeding a threshold, and (b) at least one second portion not including diagnosis-enabling data; and
display, on the display (110), a user interface including a video or audio progress bar (130) enabling a medical practitioner to navigate to specific points in time within the obtained physiological data, the user interface including at least one indication, on the video or audio progress bar (130), of a location of at least one corresponding first portion of the first portions associated with a corresponding point in time within the obtained physiological data, enabling the user to identify the location.

2. The system (100) of claim 1, wherein the physiological data is an audio or video recording and the indication includes a first marking of a start location of the at least one corresponding first portion.

3. The system (100) of claim 2, wherein the indication includes a second marking of an end location of the at least one corresponding first portion.

4. The system (100) of claim 1, wherein the physiological measurement being conducted by a user using a sensor comprised within a handheld diagnosis-device, wherein the user is not the medical practitioner, and wherein the physiological data is obtained during the physiological measurement conducted at a first geographical location and transmitted to a second geographical location of the medical practitioner, the second geographical location being remote from the first geographical location.

5. The system (100) of claim 1, wherein the processor (140) is further configured to:
receive, from the medical practitioner, an indication of an area-of-interest within the physiological data; and
send the physiological data and the indication of the area-of-interest to a remote workstation operated by a second medical practitioner, thereby enabling the remote workstation to present the physiological data and the indication of the area-of-interest to the second medical practitioner for analysis purposes.

6. The system (100) of claim 1, wherein the processor (140) is further configured to display, on the display (110), a navigation User Interface (UI) element, wherein upon activation of the navigation UI element, the system automatically navigates to a next or a previous first position of the first positions, thereby enabling skipping the second portions.

7. A method comprising:
obtaining, by a processor (140), physiological data obtained during a non-instantaneous physiological measurement of a patient, wherein the physiological data includes audio data or video data;
analyze the physiological data to identify (a) one or more first portions of the physiological data that include diagnosis-enabling data, enabling a diagnosing entity to later diagnose a medical condition of the patient, wherein the first portions having a many valued quality score exceeding a threshold, and (b)at least one second portion not including diagnosis-enabling data; and
displaying, on a display (110), by the processor (140), a user interface including a video or audio progress bar (130) enabling a medical practitioner to navigate to specific points in time within the obtained physiological data, the user interface including at least one indication, on the video or audio progress bar (130), of a location of at least one corresponding first portion of the first portions associated with a corresponding point in time within the obtained physiological data, enabling the user to identify the location.

8. The method of claim 7, wherein the physiological data is an audio or video recording and the indication includes a first marking of a start location of the at least one corresponding first portion.

9. The method of claim 8, wherein the indication includes a second marking of an end location of the at least one corresponding first portion.

10. The method of claim 7, wherein the physiological measurement being conducted by a user using a sensor comprised within a handheld diagnosis-device, wherein the user is not the medical practitioner, and wherein the physiological data is obtained during the physiological measurement conducted at a first geographical location and transmitted to a second geographical location of the medical practitioner, the second geographical location being remote from the first geographical location.

11. The method of claim 7, further comprising:
receiving, by the processor (140), from the medical practitioner, an indication of an area-of-interest within the physiological data; and
sending, by the processor (140), the physiological data and the indication of the area-of-interest to a remote workstation operated by a second medical practitioner, thereby enabling the remote workstation to present the physiological data and the indication of the area-of-interest to the second medical practitioner for analysis purposes.

12. The method of claim 7, further comprising displaying, on the display (110), a navigation User Interface (UI) element, wherein upon activation of the navigation UI element, the processor (140) automatically navigates to a next or a previous first position of the first positions, thereby enabling skipping the second portions.

13. A non-transitory computer readable storage medium having computer readable program code embodied therewith, the computer readable program code, executable by at least one processor (140) to perform a method comprising:
obtaining physiological data obtained during a non-instantaneous physiological measurement of a patient, wherein the physiological data includes audio data or video data;
analyze the physiological data to identify (a) one or more first portions of the physiological data that include diagnosis-enabling data, enabling a diagnosing entity to later diagnose a medical condition of the patient, wherein the first portions having a many valued quality score exceeding a threshold, and (b)at least one second portion not including diagnosis-enabling data; and
displaying, on a display (110), a user interface including a video or audio progress bar (130) enabling a medical practitioner to navigate to specific points in time within the obtained physiological data, the user interface including at least one indication, on the video or audio progress bar (130), of a location of at least one corresponding first portion of the first portions associated with a corresponding point in time within the obtained physiological data, enabling the user to identify the location.

## Patentansprüche

1. Ein System (100), umfassend einen Prozessor (140) und eine Anzeige (110), wobei der Prozessor (140) konfiguriert ist zum:
Erhalten physiologischer Daten, die während einer nicht-momentanen physiologischen Messung eines Patienten erhalten wurden, wobei die physiologischen Daten Audiodaten oder Videodaten beinhalten;
Analysieren der physiologischen Daten, um (a) einen oder mehrere erste Abschnitte der physiologischen Daten zu identifizieren, die diagnoseermöglichende Daten beinhalten, die es einer Diagnoseentität ermöglichen, später einen medizinischen Zustand des Patienten zu diagnostizieren, wobei die ersten Abschnitte einen vielwertigen Qualitätswert aufweisen, der einen Schwellenwert überschreitet, und (b) mindestens einen zweiten Abschnitt, der keine diagnoseermöglichenden Daten beinhaltet; und
Anzeigen, auf der Anzeige (110), einer Benutzeroberfläche, die einen Video- oder Audiofortschrittsbalken (130) beinhaltet, der medizinischem Personal ermöglicht, zu spezifischen Zeitpunkten innerhalb der erhaltenen physiologischen Daten zu navigieren, wobei die Benutzeroberfläche mindestens eine Angabe, auf dem Video- oder Audiofortschrittsbalken (130), eines Standorts von mindestens einem entsprechenden ersten Abschnitt der ersten Abschnitte beinhaltet, der mit einem entsprechenden Zeitpunkt innerhalb der erhaltenen physiologischen Daten assoziiert ist, was es dem Benutzer ermöglicht, den Standort zu identifizieren.

2. System (100) nach Anspruch 1, wobei die physiologischen Daten eine Audio- oder Videoaufzeichnung sind und die Angabe eine erste Markierung eines Startstandorts des mindestens einen entsprechenden ersten Abschnitts beinhaltet.

3. System (100) nach Anspruch 2, wobei die Angabe eine zweite Markierung eines Endstandorts des mindestens einen entsprechenden ersten Abschnitts beinhaltet.

4. System (100) nach Anspruch 1, wobei die physiologische Messung von einem Benutzer unter Verwendung eines Sensors durchgeführt wird, der in einer handgehaltenen Diagnosevorrichtung enthalten ist, wobei der Benutzer nicht das medizinische Personal ist, und wobei die physiologischen Daten während der physiologischen Messung erhalten werden, die an einem ersten geografischen Standort durchgeführt und an einen zweiten geografischen Standort des medizinischen Personals übertragen wird, wobei der zweite geografische Standort von dem ersten geografischen Standort entfernt ist.

5. System (100) nach Anspruch 1, wobei der Prozessor (140) ferner konfiguriert ist zum:
Empfangen, von dem medizinischen Personal, einer Angabe eines Bereichs von Interesse innerhalb der physiologischen Daten; und
Senden der physiologischen Daten und der Angabe des Bereichs von Interesse an eine entfernte Arbeitsstation, die von einem zweiten medizinischen Personal betrieben wird, wodurch es der entfernten Arbeitsstation ermöglicht wird, die physiologischen Daten und die Angabe des Bereichs von Interesse dem zweiten medizinischen Personal zu Analysezwecken zu präsentieren.

6. System (100) nach Anspruch 1, wobei der Prozessor (140) ferner konfiguriert ist zum Anzeigen, auf der Anzeige (110), eines Navigationsbenutzeroberflächen(Ul)-Elements, wobei bei Aktivierung des Navigations-UI-Elements das System automatisch zu einer nächsten oder einer vorherigen ersten Position der ersten Positionen navigiert, wodurch das Überspringen der zweiten Abschnitte ermöglicht wird.

7. Ein Verfahren, umfassend:
Erhalten, durch einen Prozessor (140), von physiologischen Daten, die während einer nicht-momentanen physiologischen Messung eines Patienten erhalten wurden, wobei die physiologischen Daten Audiodaten oder Videodaten beinhalten;
Analysieren der physiologischen Daten, um (a) einen oder mehrere erste Abschnitte der physiologischen Daten zu identifizieren, die diagnoseermöglichende Daten beinhalten, die es einer Diagnoseentität ermöglichen, später einen medizinischen Zustand des Patienten zu diagnostizieren, wobei die ersten Abschnitte einen vielwertigen Qualitätswert aufweisen, der einen Schwellenwert überschreitet, und (b) mindestens einen zweiten Abschnitt, der keine diagnoseermöglichenden Daten beinhaltet; und
Anzeigen, auf einer Anzeige (110), durch den Prozessor (140), einer Benutzeroberfläche, die einen Video- oder Audiofortschrittsbalken (130) beinhaltet, der medizinischem Personal ermöglicht, zu spezifischen Zeitpunkten innerhalb der erhaltenen physiologischen Daten zu navigieren, wobei die Benutzeroberfläche mindestens eine Angabe, auf dem Video- oder Audiofortschrittsbalken (130), eines Standorts von mindestens einem entsprechenden ersten Abschnitt der ersten Abschnitte beinhaltet, der mit einem entsprechenden Zeitpunkt innerhalb der erhaltenen physiologischen Daten assoziiert ist, was es dem Benutzer ermöglicht, den Standort zu identifizieren.

8. Verfahren nach Anspruch 7, wobei die physiologischen Daten eine Audio- oder Videoaufzeichnung sind und die Angabe eine erste Markierung eines Startstandorts des mindestens einen entsprechenden ersten Abschnitts beinhaltet.

9. Verfahren nach Anspruch 8, wobei die Angabe eine zweite Markierung eines Endstandorts des mindestens einen entsprechenden ersten Abschnitts beinhaltet.

10. Verfahren nach Anspruch 7, wobei die physiologische Messung von einem Benutzer unter Verwendung eines Sensors durchgeführt wird, der in einer handgehaltenen Diagnosevorrichtung enthalten ist, wobei der Benutzer nicht das medizinische Personal ist, und wobei die physiologischen Daten während der physiologischen Messung erhalten werden, die an einem ersten geografischen Standort durchgeführt und an einen zweiten geografischen Standort des medizinischen Personals übertragen wird, wobei der zweite geografische Standort von dem ersten geografischen Standort entfernt ist.

11. Verfahren nach Anspruch 7, ferner umfassend:
Empfangen, durch den Prozessor (140), von dem medizinischen Personal, einer Angabe eines Bereichs von Interesse innerhalb der physiologischen Daten; und
Senden, durch den Prozessor (140), der physiologischen Daten und der Angabe des Bereichs von Interesse an eine entfernte Arbeitsstation, die von einem zweiten medizinischen Personal betrieben wird, wodurch es der entfernten Arbeitsstation ermöglicht wird, die physiologischen Daten und die Angabe des Bereichs von Interesse dem zweiten medizinischen Personal zu Analysezwecken zu präsentieren.

12. Verfahren nach Anspruch 7, ferner umfassend Anzeigen, auf der Anzeige (110), eines Navigationsbenutzeroberflächen(UI)-Elements, wobei bei Aktivierung des Navigations-UI-Elements der Prozessor (140) automatisch zu einer nächsten oder einer vorherigen ersten Position der ersten Positionen navigiert, wodurch das Überspringen der zweiten Abschnitte ermöglicht wird.

13. Ein nicht-transitorisches computerlesbares Speichermedium mit computerlesbarem Programmcode, der damit verkörpert ist, wobei der computerlesbare Programmcode von mindestens einem Prozessor (140) ausführbar ist, um ein Verfahren durchzuführen, umfassend:
Erhalten physiologischer Daten, die während einer nicht-momentanen physiologischen Messung eines Patienten erhalten wurden, wobei die physiologischen Daten Audiodaten oder Videodaten beinhalten;
Analysieren der physiologischen Daten, um (a) einen oder mehrere erste Abschnitte der physiologischen Daten zu identifizieren, die diagnoseermöglichende Daten beinhalten, die es einer Diagnoseentität ermöglichen, später einen medizinischen Zustand des Patienten zu diagnostizieren, wobei die ersten Abschnitte einen vielwertigen Qualitätswert aufweisen, der einen Schwellenwert überschreitet, und (b) mindestens einen zweiten Abschnitt, der keine diagnoseermöglichenden Daten beinhaltet; und
Anzeigen, auf einer Anzeige (110), einer Benutzeroberfläche, die einen Video- oder Audiofortschrittsbalken (130) beinhaltet, der medizinischem Personal ermöglicht, zu spezifischen Zeitpunkten innerhalb der erhaltenen physiologischen Daten zu navigieren, wobei die Benutzeroberfläche mindestens eine Angabe, auf dem Video- oder Audiofortschrittsbalken (130), eines Standorts von mindestens einem entsprechenden ersten Abschnitt der ersten Abschnitte beinhaltet, der mit einem entsprechenden Zeitpunkt innerhalb der erhaltenen physiologischen Daten assoziiert ist, was es dem Benutzer ermöglicht, den Standort zu identifizieren.

## Revendications

1. Système (100) comprenant un processeur (140) et un écran (110), dans lequel le processeur (140) est configuré pour :
obtenir des données physiologiques obtenues lors d'une mesure physiologique non instantanée d'un patient, dans lesquelles les données physiologiques comprennent des données audio ou des données vidéo ;
analyser les données physiologiques afin d'identifier (a) une ou plusieurs premières parties des données physiologiques qui comprennent des données d'activation de diagnostic, permettant à une entité de diagnostic de diagnostiquer ultérieurement un problème de santé du patient, dans lesquelles les premières parties ont un score de qualité à valeurs multiples dépassant un seuil, et (b) au moins une deuxième partie ne comprenant pas de données d'activation de diagnostic ; et
afficher, sur l'écran (110), une interface utilisateur comprenant une barre de progression vidéo ou audio (130) permettant à un médecin praticien de naviguer vers des points spécifiques dans le temps au sein des données physiologiques obtenues, l'interface utilisateur comprenant au moins une indication, sur la barre de progression vidéo ou audio (130), d'un emplacement d'au moins une première partie correspondante des premières parties associées à un point correspondant dans le temps au sein des données physiologiques obtenues, permettant à l'utilisateur d'identifier l'emplacement.

2. Système (100) selon la revendication 1, dans lequel les données physiologiques sont un enregistrement audio ou vidéo et l'indication comprend un premier marquage d'un emplacement de début de l'au moins une première partie correspondante.

3. Système (100) selon la revendication 2, dans lequel l'indication comprend un deuxième marquage d'un emplacement de fin de l'au moins une première partie correspondante.

4. Système (100) selon la revendication 1, dans lequel la mesure physiologique est effectuée par un utilisateur à l'aide d'un capteur compris dans un dispositif de diagnostic portatif, dans lequel l'utilisateur n'est pas le médecin praticien, et dans lequel les données physiologiques sont obtenues pendant la mesure physiologique effectuée à un premier emplacement géographique, et sont transmises à un deuxième emplacement géographique du médecin praticien, le deuxième emplacement géographique étant éloigné du premier emplacement géographique.

5. Système (100) selon la revendication 1, dans lequel le processeur (140) est en outre configuré pour :
recevoir, en provenance du médecin praticien, une indication d'une zone d'intérêt au sein des données physiologiques ; et
envoyer les données physiologiques et l'indication de la zone d'intérêt à un poste de travail distant exploité par un deuxième médecin praticien, ce qui permet au poste de travail distant de présenter les données physiologiques et l'indication de la zone d'intérêt au deuxième médecin praticien à des fins d'analyse.

6. Système (100) selon la revendication 1, dans lequel le processeur (140) est en outre configuré pour afficher, sur l'écran (110), un élément d'interface utilisateur (UI) de navigation, dans lequel, lors de l'activation de l'élément UI de navigation, le système navigue automatiquement vers une première position suivante ou précédente parmi les premières positions, ce qui permet de sauter les deuxièmes parties.

7. Procédé comprenant le fait de :
obtenir, par le biais d'un processeur (140), des données physiologiques obtenues lors d'une mesure physiologique non instantanée d'un patient, dans lesquelles les données physiologiques comprennent des données audio ou des données vidéo ;
analyser les données physiologiques afin d'identifier (a) une ou plusieurs premières parties des données physiologiques qui comprennent des données d'activation de diagnostic, permettant à une entité de diagnostic de diagnostiquer ultérieurement un problème de santé du patient, dans lesquelles les premières parties ont un score de qualité à valeurs multiples dépassant un seuil, et (b) au moins une deuxième partie ne comprenant pas de données d'activation de diagnostic ; et
afficher, sur l'écran (110), par le biais du processeur (140), une interface utilisateur comprenant une barre de progression vidéo ou audio (130) permettant à un médecin praticien de naviguer vers des points spécifiques dans le temps au sein des données physiologiques obtenues, l'interface utilisateur comprenant au moins une indication, sur la barre de progression vidéo ou audio (130), d'un emplacement d'au moins une première partie correspondante des premières parties associées à un point correspondant dans le temps au sein des données physiologiques obtenues, permettant à l'utilisateur d'identifier l'emplacement.

8. Procédé selon la revendication 7, dans lequel les données physiologiques sont un enregistrement audio ou vidéo et l'indication comprend un premier marquage d'un emplacement de début de l'au moins une première partie correspondante.

9. Procédé selon la revendication 8, dans lequel l'indication comprend un deuxième marquage d'un emplacement de fin de l'au moins une première partie correspondante.

10. Procédé selon la revendication 7, dans lequel la mesure physiologique est effectuée par un utilisateur à l'aide d'un capteur compris dans un dispositif de diagnostic portatif, dans lequel l'utilisateur n'est pas le médecin praticien, et dans lequel les données physiologiques sont obtenues pendant la mesure physiologique effectuée à un premier emplacement géographique, et sont transmises à un deuxième emplacement géographique du médecin praticien, le deuxième emplacement géographique étant éloigné du premier emplacement géographique.

11. Procédé selon la revendication 7, comprenant en outre le fait de :
recevoir, par le biais du processeur (140), en provenance du médecin praticien, une indication d'une zone d'intérêt au sein des données physiologiques ; et
envoyer, par le biais du processeur (140), les données physiologiques et l'indication de la zone d'intérêt à un poste de travail distant exploité par un deuxième médecin praticien, ce qui permet au poste de travail distant de présenter les données physiologiques et l'indication de la zone d'intérêt au deuxième médecin praticien à des fins d'analyse.

12. Procédé selon la revendication 7, comprenant en outre le fait d'afficher, sur l'écran (110), un élément d'interface utilisateur (UI) de navigation, dans lequel, lors de l'activation de l'élément UI de navigation, le processeur (140) navigue automatiquement vers une première position suivante ou précédente parmi les premières positions, ce qui permet de sauter les deuxièmes parties.

13. Support de stockage non transitoire lisible par ordinateur ayant un code de programme lisible par ordinateur incorporé dans celui-ci, le code de programme lisible par ordinateur étant exécutable par au moins un processeur (140) pour mettre en œuvre un procédé comprenant le fait de :
obtenir des données physiologiques obtenues lors d'une mesure physiologique non instantanée d'un patient, dans lesquelles les données physiologiques comprennent des données audio ou des données vidéo ;
analyser les données physiologiques afin d'identifier (a) une ou plusieurs premières parties des données physiologiques qui comprennent des données d'activation de diagnostic, permettant à une entité de diagnostic de diagnostiquer ultérieurement un problème de santé du patient, dans lesquelles les premières parties ont un score de qualité à valeurs multiples dépassant un seuil, et (b) au moins une deuxième partie ne comprenant pas de données d'activation de diagnostic ; et
afficher, sur un écran (110), une interface utilisateur comprenant une barre de progression vidéo ou audio (130) permettant à un médecin praticien de naviguer vers des points spécifiques dans le temps au sein des données physiologiques obtenues, l'interface utilisateur comprenant au moins une indication, sur la barre de progression vidéo ou audio (130), d'un emplacement d'au moins une première partie correspondante des premières parties associées à un point correspondant dans le temps au sein des données physiologiques obtenues, permettant à l'utilisateur d'identifier l'emplacement.
